# EUROPEAN PATENT APPLICATION

(11) **EP 1 710 246 A1**
(43) Date of publication of application: **11.10.2006**
(21) Application number: 05090098.4
(22) Date of filing: 08.04.2005
(51) Int. Cl.: C07D 513/08, A61K 31/529

(54) **Sulfoximine-pyrimidine Macrocycles and the salts thereof, a process for making them, and their pharmaceutical use against cancer**

(71) Applicant: Schering Aktiengesellschaft, 13353 Berlin (DE)
(72) Inventor: Luecking, Ulrich, 10245 Berlin (DE)

(57) **Abstract**

The invention relates to macrocyclic sulfoximines according to the general Formula I: 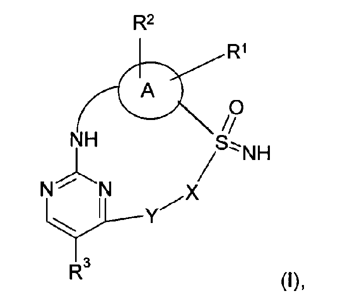 wherein A, X, Y, R¹, R² and R³ have the meaning as given in the specification and the claims and the salts thereof; to pharmaceutical compositions comprising the macrocyclic sulfoximines and to a method of preparing the macrocyclic sulfoximines as well as the use thereof for manufacturing a pharmaceutical composition for the treatment of diseases of dysregulated vascular growth or of diseases which are accompanied with dysregulated vascular growth, wherein the compounds effectively interfere with angiopoietin and therefore influence Tie2 signalling.

## Description

The invention relates to macrocyclic sulfoximines and the salts thereof, to pharmaceutical compositions comprising the macrocyclic sulfoximines and to a method of preparing the macrocyclic sulfoximines as well as the use thereof.

In order to defeat diseases with dysregulated vascular growth like cancer different strategies were developed. One possible strategy is the blockade of angiogenesis to the tumour tissue, because tumour angiogenesis is a prerequisite for the growth of solid tumours.

The angiogenesis represents beside the vasculogenesis one of two basic processes during the genesis of vasculature. Vasculogenesis names the neoplasm of vasculature during the embryo development, wherein the angiogenesis describes the neoplasm of vasculature by sprouts or division of present vasculature. It has been found that two receptors expressed on endothelial cells, VEGF- (vascular endothelial growth factor) and Tie-receptors, are essential for normal development of vascular tissue as blood vessels (Dumont et al., (1994). Dominant-negative and targeted null mutations in the endothelial receptor tyrosine kinase, tek, reveal a critical role in vasculogenesis of the embryo. Genes Dev, 8:1897-909; Sato et al.: "Distinct roles of the receptor tyrosine kinases Tie-1 and Tie-2 in blood vessel formation" Nature. 1995, Jul 6; 376(6535):70-4.).

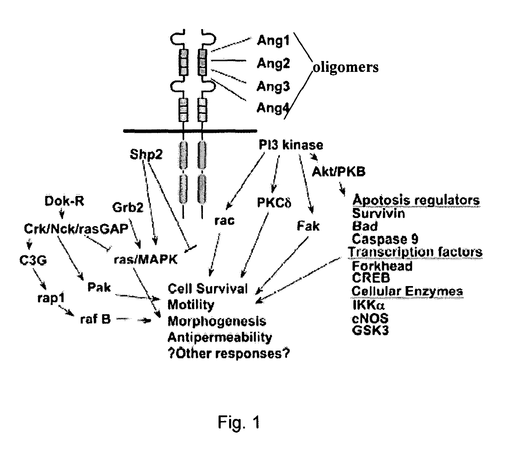

The mechanism of Tie2 signalling was characterized by different searchers, wherein different angiopoietins were found to be involved. So it could be explained that angiopoietin-1 if bound to the extracellular domain of the Tie2-receptor stimulates autophosphorylation and activates the intracellular kinase domain. Angiopoietin-1 activation of Tie2 however does not stimulate mitogenesis but rather migration. Angiopoietin-2 can block angiopoietin-1 mediated Tie2 activation and the resulting endothelial migration. This indicates that angiopoietin-2 is a naturally occurring inhibitor of Tie2 activation (Maisonpierre et al.: "Angiopoietin-2, a natural antagonist for Tie2 that disrupts in vivo angiogenesis". Science. 1997, Jul 4; 277(5322):55-60; Witzenbichler et al.: "Chemotactic properties of angiopoietin-1 and -2, ligands for the endothelial-specific receptor tyrosine kinase Tie2". J Biol Chem. 1998, Jul 17; 273(29):18514-21). For an overview see Figure 1 modified by Peters *et al.* (Peters et al.: "Functional significance of Tie2 signalling in the adult vasculature". Recent Prog Horm Res. 2004; 59:51-71. Review.).

Receptor dimerization results in cross-phosphorylation on specific tyrosine-residues. Receptor cross-phosphorylation has a dual effect: it enhances the receptor's kinase activity and it provides binding sites for signalling molecules possessing phosphotyrosine binding domains (SH2 and PTB domains) (Pawson T.: "Regulation and targets of receptor tyrosine kinases". Eur J Cancer. 2002, Sep, 38 Suppl 5:S3-10. Review).

The signalling cross-talk between the PI3-K pathway and the Dok-R pathway is required for an optimal chemotactic response downstream of Tie2. Other recent studies have shown that Tie2-mediated activation of the P13-K/Akt pathway is required for endothelial nitric oxide synthase (eNOS) activation, focal adhesion kinase activation, and protease secretion, all of which may contribute importantly to Tie2 function during angiogenesis (Kim l. et al.: "Angiopoietin-1 regulates endothelial cell survival through the phosphatidylinositol 3'-Kinase/Akt signal transduction pathway". Circ Res. 2000, Jan 7-21; 86(1):24-9; Babaei et al.: "Angiogenic actions of angiopoietin-1 require endothelium-derived nitric oxide". Am J Pathol. 2003, Jun; 162(6):1927-36).

For normal development a balanced interaction between the receptors and socalled ligands is necessary. Especially the angiopoietins, which signal via Tie2 receptors, play an important role in angiogenesis (Babaei *et al.,* **2003**).

The broad expression of Tie2 in adult vasculature has been confirmed in transgenic mice using Tie2 promoter driven reporters (Schlaeger et al.: "Uniform vascular-endothelial-cell-specific gene expression in both embryonic and adult transgenic mice". Proc Natl Acad Sci USA. 1997, Apr 1; 94(7):3058-63; Motoike et al.: "Universal GFP reporter for the study of vascular development". Genesis. 2000, Oct; 28(2):75-81). Immunohistochemical analysis demonstrated the expression of Tie2 in adult rat tissues undergoing angiogenesis. During ovarian folliculogenesis, Tie2 was expressed in the neo-vessels of the developing corpus luteum. Angiopoietin-1 and angiopoietin-2 also were expressed in the corpus luteum, with angiopoietin-2 localizing to the leading edge of proliferating vessels and angiopoietin-1 localizing diffusely behind the leading edge (Maisonpierre *et al.,* 1997). It was suggested that angiopoietin-2-mediated inhibition of Tie2 activation serves to "destabilize" the vessel, to make it responsive to other angiogenic growth factors such as VEGF. Subsequently, angiopoietin-1-mediated activation of Tie2 would trigger stabilization of the neovasculature.

The disruption of Tie2 function shows the relevance of Tie2 for neoangiogenesis in transgenic mice resulting in early embryonic lethality as a consequence of vascular abnormalities (Dumont *et al.,* **1994;** Sato *et al.,* **1995).** Tie2-/- embryos failed to develop the normal vessel hierarchy, suggestive of a failure of vascular branching and differentiation. Tie2-/- embryos have a decreased number of endothelial cells and furthermore less contact between endothelial cells and the underlying pericytes/smooth muscle cells. This implies a role in the maturation and stabilization of newly formed vasculature.

The studies in mice with transgenic or ablated Tie2 gene suggest a critical role for Tie2 in maturation of vascular development in embryos and in adult vasculature. Conditional expression of Tie2 in the endothelium of mice homozygous for a Tie2 null allele partially rescued the embryonic lethality of the Tie2 null phenotype (Jones N et al.: "Tie receptors: new modulators of angiogenic and lymphangiogenic responses." Nat Rev Mol Cell Biol. 2001 Apr; 2(4):257-67. Review). Mice lacking functional angiopoietin-1 expression and mice over-expressing angiopoietin-2 both displayed a phenotype similar to Tie2-/- mice (Suri et al.: "Requisite role of angiopoietin-1, a ligand for the TIE2 receptor, during embryonic angiogenesis." Cell. 1996 Dec 27; 87(7): 1171-80; Maisonpierre PC et al.: "Angiopoietin-2, a natural antagonist for Tie2 that disrupts in vivo angiogenesis. Science. 1997 Jul 4; 277(5322):55-60.).

Angiopoietin-2 -/- mice have profound defects in the growth and patterning of lymphatic vasculature and fail to remodel and regress the hyaloid vasculature of the neonatal lens (Gale et al.: "Angiopoietin 2 is required for postnatal angiogenesis and lymphatic patterning, and only the latter role is rescued by Angiopoietin-1". Dev Cell. 2002, Sep; 3(3):411-23). Angiopoietin-1 rescued the lymphatic defects, but not the vascular remodeling defects. So angiopoietin-2 might function as a Tie2 antagonist in blood vasculature but as a Tie2 agonist in developing lymph vasculature.

Tie2 also plays a role in pathological angiogenesis. It was shown that mutations in Tie2 cause inherited venous malformations and enhance both ligand dependent and independent Tie2 kinase activity (Vikkula et al.: "Dysmorphogenesis caused by an activating mutation in the receptor tyrosine kinase TIE2". Cell. 1996, Dec 27; 87(7):1181-90). Tie2 expression was investigated in human breast cancer tumour specimens and Tie2 expression was found in the vascular endothelium both in normal breast tissue and in breast tumours. The proportion of Tie2-positive tumour microvessels was increased in tumours as compared to normal breast tissue (Peters KG et al.: "Expression of Tie2/Tek in breast tumour vasculature provides a new marker for evaluation of tumour angiogenesis. BrJ Cancer. 1998, 77(1):51-6).

Angiopoietin-1 overexpression in tumour models resulted in decreased tumour growth. The effect is possibly related to angiopoietin-1 mediated stabilization of the tumour vasculature, which renders the vessels resistant to angiogenic stimuli (Hayes et a/.: "Expression and function of angiopoietin-1 in breast cancer". BrJ Cancer. 2000, Nov; 83(9):1154-60; Shim et al.: "Inhibition of angiopoietin-1 expression in tumour cells by an antisense RNA approach inhibited xenograft tumour growth in immunodeficient mice". Int J Cancer. 2001, Oct 1; 94(1):6-15; Shim et al.: "Angiopoietin 1 promotes tumour angiogenesis and tumour vessel plasticity of human cervical cancer in mice". Exp Cell Res. 2002, Oct 1; 279(2):299-309; Hawighorst et al.: "Activation of the tie2 receptor by angiopoietin-1 enhances tumour vessel maturation and impairs squamous cell carcinoma growth". Am J Pathol. 2002, Apr;160(4):1381-92.; Stoeltzing et al.: "Angiopoietin-1 inhibits vascular permeability, angiogenesis, and growth of hepatic colon cancer tumours". Cancer Res. 2003, Jun 15; 63(12):3370-7.).

Corneal angiogenesis induced by tumour cell conditioned medium was inhibited by recombinant sTie, despite the presence of VEGF. Mammary tumour growth was significantly inhibited in a skin chamber tumour model recombinant sTie2 (Lin et al.: "Inhibition of tumour angiogenesis using a soluble receptor establishes a role for Tie2 in pathologic vascular growth". J Clin Invest. 1997, Oct 15;100(8):2072-8; Lin et al.: "Antiangiogenic gene therapy targeting the endothelium-specific receptor tyrosine kinase Tie2". Proc Natl Acad Sci U S A. 1998, Jul 21; 95(15):8829-34). Similar sTie constructs have shown comparable effects in different tumour models (Siemeister et al.: "Two independent mechanisms essential for tumour angiogenesis: inhibition of human melanoma xenograft growth by interfering with either the vascular endothelial growth factor receptor pathway or the Tie-2 pathway". Cancer Res. 1999, Jul 1; 59(13):3185-91; Stratmann et al.: "Differential inhibition of tumour angiogenesis by tie2 and vascular endothelial growth factor receptor-2 dominant-negative receptor mutants". Int J Cancer. 2001, Feb 1; 91 (3):273-82; Tanaka et al.: "Tie2 vascular endothelial receptor expression and function in hepatocellular carcinoma". Hepatology. 2002, Apr; 35(4):861-7).

When the interaction of angiopoietin-2 with its receptor is blocked by application of a neutralizing anti-angiopoietin-2 monoclonal antibody, the growth of experimental tumours can be blocked efficiently again pointing to the important role of Tie2 in tumour angiogenesis and growth (Oliner et al.: "Suppression of angiogenesis and tumour growth by selective inhibition of angiopoietin-2". Cancer Cell. 2004, Nov; 6(5):507-16.) So inhibiting the Tie2 pathway will inhibit pathological angiogenesis.

To influence the interaction between receptor and ligand it could be shown that angiogenesis may be blocked with blockers like Avastin which interfere with VEGF signal transduction to endothelial cells.

Avastin is a clinically effective antibody that functions as tumour growth inhibitor by blockade of VEGFR mediated angiogenic signalling. Thus interference with VEGF signalling is a proven clinical principle. VEGF-C is a molecule inducing lymph angiogenesis via VEGFR 3. The blockade of this signal pathway is inhibiting diseases associated with lymph angiogenesis as is lymphedema and related diseases (Saharinen et al.: "Lymphatic vasculature: development, molecular regulation and role in tumour metastasis and inflammation." Trends Immunol. 2004, Jul:25(7): 387-95. Review).

Pyrimidines and their derivatives have been frequently described as therapeutic agents for diverse diseases. A series of recently published patent applications describes their use as inhibitors of various protein kinases, for example WO 2003/032994 A, WO 2003/063794 A, WO 2003/076437 A and WO 2002/096888 A. More specifically, certain pyrimidine derivatives have been disclosed as inhibitors of protein kinases involved in angiogenesis, such as VEGF or Tie2, for example benzimidazole substituted 2,4-diaminopyrimidines (WO 2003/074515 A) or (bis)anilino-pyrimidines (WO 2003/066601 A). Very recently, pyrimidine derivatives in which the pyrimidine constitutes a part of a macrocyclic ring system have been reported to be inhibitors of CDKs and/or VEGF (WO 2004/026881 A), or of CDK2 and/or CDK5, respectively (WO 2004/078682 A).

A particular problem in using such known substances as inhibitors or blockers is that their use at the same time is often accompanied with undesired cytotoxic side effects on normal developing and proliferating tissue. This originates from substances which are less selective and at the same time dose tolerability problems.

Therefore the aim of the present invention is to provide compounds, which are useful for the treatment of diseases of dysregulated vascular growth or diseases which are accompanied by dysregulated vascular growth. Furthermore the prior art problems shall be prevented, especially compounds shall be provided, which show low toxic side effects on normal proliferating tissue but are effectively inhibiting endothelial cell migration at small concentrations. This will further reduce undesired side effects.

The solution to the above problems is achieved by providing compounds derived from a class of macrocyclic sulfoximines (sulfoximine-macrocycles) and the, solvates, hydrates, N-oxides, isomers and salts thereof in accordance with claim 1, the method of preparing sulfoximine-macrocycles in accordance with claim 24, a pharmaceutical composition in accordance with claim 25, the use of the compounds in accordance with claim 26 and a method for treating diseases with the compounds in accordance with claim 33.

The invention relates to a compound of the general Formula 1: 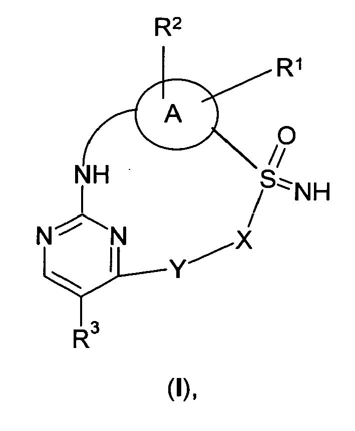 in which
- A: is -phenylene- or -C₅-C₆-heteroarylene-;
- X: is a 2- to 6-membered alkylene tether which is unsubstituted or singly or multiply substituted by substituents selected from the group comprising halogen, C₁-C₄-alkoxy, hydroxy, amino, cyano, carboxy, -NH-C₁-C₄-alkyl, -N(C₁-C₄-alkyl)₂, and C₁-C₄-alkyl, which itself may be unsubstituted or singly or multiply substituted by halogen, C₁-C₄-alkoxy, hydroxy, amino, cyano, carboxy, -NH-C₁-C₄-alkyl, or -N(C₁-C₄-alkyl)₂;
- Y: is -NR⁴-, -O-,or -S-;
- R¹ and R²: are the same or different and are independently from each other selected from the group comprising hydrogen, hydroxy, halogen, nitro, cyano, -(CH₂)pPO₃(R⁵)₂, -(CH₂)ₚ-NR⁵R⁶, -(CH₂)ₚ-NR ⁵COR ⁶, -(CH₂)ₚ-NR⁵CSR⁶, -(CH₂)ₚ-NR^{s}S(0)R⁶, -(CH₂)ₚ-NR⁵S(O)₂R⁶, -(CH₂)ₚ-NR⁵CONR⁶R⁷, -(CH₂)ₚ-NR ⁵COOR ⁶, -(CH₂)ₚ-NR⁵C(NH)NR⁶R⁷, -(CH₂)ₚ-NRsCSNR⁶R⁷, -(CH₂)ₚ-NR⁵S(O)NR⁶R 7, -(CH₂)ₚ-NR ⁵S(O)₂NR⁶R⁷, -(CH₂)ₚ-COR⁵, -(CH₂)ₚ-CR (OH)-R , -(CH₂)ₚ-CSR , -(CH₂)ₚ-S(O)R⁵ , -(CH₂)ₚ-S(O)(NH)R⁵, -(CH₂)ₚ-S(O)₂R⁵, -(CH₂)ₚ-S(O)₂NR⁵R⁶, -(CH₂)ₚ-S(O)₂N=CH-NR⁵R⁶, -(CH₂)ₚ-SO₃R⁵, -(CH₂)ₚ-CO₂R , -(CH₂)ₚ-CONR⁵R⁶, -(CH₂)ₚ-CSNR⁵R⁶, -(CH₂)ₚ-SR⁵, -(CH₂)ₚ-OR⁵, -CR⁵(OH)-R⁶, -O-(CH₂)ₚ-COR⁵, -O-(CH₂)ₚ-CR⁴(OH)-R⁵, and moieties being selected from the group comprising -C₁-C₈-alkyl, -C₂-C₈-alkenyl, -C₂-C₈-alkynyl, -C₃-C₈-cycloalkyl, -C₃-C₈-heterocydoalkyl, -(CH₂)_{P}-phenyl, -(CH₂)ₚ-C₅-C₆-heteroaryl, -phenylene-(CH₂)ₚ-R⁵,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl, and wherein 0 to 2 methylene groups of -C₁-C₈-alkyl may be replaced independently of each other by -O-, -S-, -C(=O)-, -SO₂- or -NR⁴-;
- R³: is selected from the group comprising halogen, nitro, cyano, -C₁₋C₆-alkylthio, amino,
-NH-(CH₂)ₚ-C₃-C₈-Cycloalkyl, benzodioxolyl, -(CH₂)ₚPO₃(R⁵)₂, -NR⁵R⁶, -S(O)(C₁-C₆-alkyl), -S(O)₂(C₁-C₆-alkyl), -CONR⁵R⁶, -COR⁵, -COOR⁵, -NR⁵-(CH₂)ₚ-NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷,
and moieties, said moieties being selected from the group comprising -C₁-C₈-alkyl, -C₂-C₈-alkenyl, -C₂-C₈-alkynyl, -C₃-C₈-cydoalkyl, -C₃-C₈-heterocycloalkyl, -(CH₂)ₚ-phenyl, -(CH₂)ₚ-C₅-C₁₀-heteroaryl, -O-phenyl, -O-C₅-C₁₀-heteroaryl, -phenylene-(CH₂)ₚ-R⁵
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -NH-(CH₂)ₚ-C₃-C₈-cycloalkyl, -C₃-C₈-cycloalkyl, -C₃-C₈-heterocydoalkyl, -(CH₂)ₚ-phenyl, -(CH₂)ₚ-C₅-C₆-heteroaryl, -phenylene-(CH₂)ₚ-R⁵, -NR⁵R⁶, -C₁-C₆-hydroxyalkyl, -C₁-C₆-haloalkyl, -C₁-C₆-haloalkoxy, -C₂-C₆-alkenyl, -C₂-C₆-alkynyl, -C₁-C₆-alkoxy-C₁-C₆-alkyl, -C₁-C₆-alkoxy-C₁-C₆-alkoxy-C₁-C₆-alkyl, -(CH₂)ₚPO₃(R⁵)₂, -(CH₂)ₚ-NR⁵CONR⁶R⁷, -(CH₂)ₚ-S(O)R⁵, -(CH₂)ₚ-S(O)₂R⁵, -(CH₂)ₚ-NR⁵S(O)₂R⁶, -(CH₂)ₚ-S(O)₂NR⁵R⁶, -(CH₂)_{P}-CONR⁵R⁶, -(CH₂)ₚ-NR⁵COR⁶, -(CH₂)ₚ-CR⁴(OH)-R⁵, -O-(CH₂)ₚ-CR⁴(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein phenylene, -C₃-C₈-cydoalkyl, -C₃-C₈-heterocydoalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃, and wherein 0 to 2 methylene groups of -C₁-C₈-alkyl may be replaced independently of each other by -O-, -S-, -C(=O)-, -SO₂- or-NR⁴-;
- R⁴: is hydrogen or -C₁-C₈-alkyl;
- R⁵, R⁶,:
- R⁷ and R⁸: are the same or different and are independently from each other selected from the group comprising hydrogen, benzodioxolyl and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocydoalkyl, phenyl, -C₅-C₁₀-heteroaryl,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -NH-(CH₂)ₚ-C₃-C₈-cycloalkyl, -C₃-C₈-cycloalkyl, -C₃-C₈-heterocycloalkyl, -C₁-C₆-hydroxyalkyl, -C₁-C₆-haloalkyl, -C₁-C₆-haloalkoxy, -C₂-C₆-alkenyl, -C₂-C₆-alkynyl, -C₁-C₆-alkoxy-C₁-C₆-alkyl, -C₁-C₆-alkoxy-C₁-C₆-alkoxy-C₁-C₆-alkyl, -NR⁹R¹⁰, -NR⁹CONR¹⁰, -S(O)(C₁-C₆-alkyl), -S(O)₂(C₁-C₆-alkyl), -S(O)₂-phenyl, -NH-S(O)₂(C₁-C₆-alkyl), -NH-S(O)₂-phenyl, -S(O)₂-NH-(C₁-C₆-alkyl), -S(O)₂-NH-phenyl, -C₁-C₆-alkanoyl, -CONR⁹R¹⁰, -COR⁹, -COOR⁹, -(CH₂)ₚ-phenyl, -(CH₂)ₚ-C₅-C₁₀-heteroaryl, -phenylene-(CH₂)ₚ-R⁹, -(CH₂)_{P}PO₃(R⁹)₂, wherein phenylene, -C₃-C₈-cydoalkyl, -C₃-C₈-heterocycloalkyl, phenyl, -(CH₂)ₚ-phenyl and -(CH₂)ₚ-C₅-C₁₀-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or -NR⁴-;
or
two members selected from the group comprising R⁵, R⁶, R⁷ and R⁸ form a 2- to 8-membered alkylene tether which may be unsubstituted or singly substituted by -(CH₂)ₚ-OH, -(CH₂)ₚ-CN, or -(CH₂)ₚ-NR⁹R¹⁰, and in which 0 to 2 methylene groups may be replaced by -O-, -S-, -(C=O)-, -S(O)₂-, or -NR⁴-;
- R⁹, R¹⁰: are independently from each other hydrogen or -C₁-C₈-alkyl;
- p: is from 0 - 6;
and solvates, hydrates, N-oxides, isomers and salts thereof.

The following terms as mentioned herein and in the claims have preferably the following meanings:

The term alkyl preferably refers to branched and unbranched alkyl, meaning e.g. methyl, ethyl, *n*-propyl, iso-propyl, *n*-butyl, iso-butyl, *tert*-butyl, sec-butyl, pentyl, iso-pentyl, hexyl, heptyl, octyl, nonyl and decyl and the isomers thereof.

The term haloalkyl preferably refers to branched and unbranched alkyl being substituted at least by one halogen atom as described below, e.g. chloromethyl, fluoropropyl, difluoromethyl, trichloromethyl, 2,2,2-trifluoroethyl, bromobutyl, trifluoromethyl, iodoethyl, and isomers thereof.

The term hydroxyalkyl preferably refers to branched and unbranched alkyl, being substituted by at least one hydroxy group, e.g. hydroxymethyl, hydroxyethyl, 2-hydroxypropyl, 3-hydroxypropyl, 4-hydroxybutyl, 5-hydroxyhexyl, 3,4-dihydroxybutyl, and isomers thereof.

The term alkoxy preferably refers to branched and unbranched alkoxy, meaning e.g. methoxy, ethoxy, propyloxy, iso-propyloxy, butyloxy, iso-butyloxy, *tert-*butyloxy, sec-butyloxy, pentyloxy, iso-pentyloxy, hexyloxy, heptyloxy, octyloxy, nonyloxy, decyloxy, undecyloxy and dodecyloxy and the isomers thereof.

The term haloalkoxy preferably refers to branched and unbranched alkoxy being substituted by at least one halogen atom as described below, e.g. chloromethoxy, fluoropropyloxy, difluoromethyloxy, trichloromethoxy, 2,2,2-trifluoroethoxy, bromobutyloxy, trifluoromethoxy, iodoethoxy, and isomers thereof.

The term cycloalkyl preferably refers to saturated cycloalkyl groups of the indicated ring size, meaning e.g. cyclopropyl, cyclobutyl, cyclopentyl, cyclohexyl and cycloheptyl, and also to unsaturated cycloalkyls containing one or more double bonds in the C-backbone, e.g. to cycloalkenyl, such as cyclopropenyl, cyclobutenyl, cyclopentenyl, cyclohexenyl and cycloheptenyl, wherein the linkage can be provided to the double or single bond.

The term heterocycloalkyl refers to cyloalkyl groups as described above featuring the indicated number of ring atoms wherein one or more ring atoms are heteroatoms such as nitrogen, oxygen or sulphur, or carbonyl groups, or - otherwise stated - in a Cₙ-cycloalkyl group one or more carbon atoms are replaced by these heteroatoms to give such Cₙ cycloheteroalkyl group. Thus such group refers e.g. to a three-membered heterocycloalkyl, expressed as -C₃₋heterocycloalkyl such as oxyranyl. Other examples of heterocycloalkyls are oxetanyl (C₄), aziridinyl (C₃), azetidinyl (C₄), tetrahydrofuranyl (C₅), pyrrolidinyl (C₅), morpholinyl (C₆), dithianyl (C₆), thiomorpholinyl (C₆), piperazinyl (C₆), trithianyl (C₆) and chinuclidinyl (C₈).

Halogen or Hal preferably refers to fluorine, chlorine, bromine and iodine.

The term alkenyl preferably refers to branched and unbranched alkenyl, e.g. to vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl and 2-methyl-prop-1-en-1-yl.

The term alkynyl preferably refers to branched and unbranched alkynyl, e.g. to ethynyl, prop-1-yn-1-yl, but-1-yn-1-yl, but-2-yn-1-yl and but-3-yn-1-yl.

The term aryl refers to monocyclic or polycyclic aromatic groups featuring the indicated number of ring atoms, e.g. to phenyl, naphthyl and biphenyl.

The term heteroaryl refers to monocyclic or polycyclic aromatics featuring the indicated number of ring atoms wherein one or more ring atoms are heteroatoms such as nitrogen, oxygen or sulphur or - otherwise stated - in a Cₙ-aryl group one or more carbon atoms are replaced by these heteroatoms to give such Cₙ-heteroaryl group. The term heteroaryl may be exemplified by but is not limited to e.g. to five-membered heteroaryl, expressed as -C₅-heteroaryl, such as thiophenyl, furanyl, oxazolyl, thiazolyl, imidazolyl, pyrazolyl, triazolyl, thia-4H-pyrazolyl; or six-membered heteroaryl, expressed as -C₆-heteroaryl, such as pyridinyl, pyrimidinyl, triazinyl. The term heteroaryl may be furthermore exemplified but is not limited to benzo-derivatives of said -C₅- and -C₆₋heteroaryls, such as indolyl, benzofuranyl, or benzimidazolyl, expressed as -C₉₋heteroaryl, or quinolinyl and isoquinolinyl, expressed as -C₁₀-heteroaryl.

The term arylene refers to monocyclic or polycyclic arylene aromatic systems featuring the indicated number of ring atoms, e.g. to phenylene, naphthylene and biphenylene. If the term phenylene is used it should be understood that the linking residues can be arranged to each other in ortho-, para- or meta-position.

The term heteroarylene refers to monocyclic or polycyclic arylenes featuring the indicated number of ring atoms wherein one or more ring atoms are heteroatoms such as nitrogen, oxygen or sulphur or - otherwise stated - in a Cₙ-arylene group one or more carbon atoms are replaced by these heteroatoms to give such Cₙ-heteroarylene group. The term heteroarylene may be exemplified by but is not limited to e.g. to five-membered heteroarylene, expressed as -C₅-heteroarylene, such as thiophenylene, furanylene, oxazolylene, thiazolylene, imidazolylene, pyrazolylene, triazolylene, thia-4H-pyrazolylene; or six-membered heteroarylene, expressed as -C₆-heteroarylene, such as pyridinylene, pyrimidinylene, triazinylene, and benzo-derivates thereof such as quinolinylene and isoquinolinylene.
The term heteroarylene may be furthermore exemplified but is not limited to benzo-derivatives of said -C₅- and -C₆-heteroarylenes, such as indolylene, benzofuranylene, or benzimidazolylene, expressed as -C₉-heteroarylene; or quinolinylene and isoquinolinylene, expressed as -C₁₀-heteroaryl.

The compound according to Formula I (sulfoximine-macrocycles) can exist as N-oxides which are defined in that at least one nitrogen of the compounds of the general Formula I may be oxidized.

The compound according to Formula I (sulfoximine-macrocycles) can exist as a solvate, in particular as a hydrate, wherein the compound according to Formula I may contain polar solvents, in particular water, as structural element of the crystal lattice of the compounds. The amount of polar solvents, in particular water, may exist in a stoichiometric or unstoichiometric ratio. In case of stoichiometric solvates, e.g. hydrate, hemi-, (semi-), mono-, sesqui-, di-, tri-, tetra-, penta- etc. solvates or hydrates, respectively, are possible.

The term isomers refers to chemical compounds with the same number and types of atoms as another chemical species. There are two main classes of isomers, constitutional isomers and stereoisomers.

The term constitutional isomers refers to chemical compounds with the same number and types of atoms, but they are connected in differing sequences. There are functional isomers, structural isomers, tautomers or valence isomers.

In stereoisomers, the atoms are connected sequentially in the same way, such that condensed Formulae for two isomeric molecules are identical. The isomers differ, however, in the way the atoms are arranged in space. There are two major sub-classes of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers. Diastereomers, which still have a different constitution, are another sub-class of diastereomers, the best known of which are simple *cis - trans* isomers.

In order to limit different types of isomers from each other reference is made to IUPAC Roles Section E *(*Pure Appl Chem 45, 11-30, 1976).

The compound according to Formula I (sulfoximine-macrocycles) can exist in free form or in a salt form, wherein the salt can be a suitable pharmaceutically acceptable salt. A suitable pharmaceutically acceptable salt of the sulfoximine-macrocycles of the invention is, for example, an acid-addition salt of a sulfoximine-macrocycle of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a sulfoximine-macrocycle of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glukamine, dimethyl-glukamine, ethyl-glukamine, lysine, 1,6-hexadiamin, ethanolamine, glucosamine, sarkosine, serinole, tris-hydroxy-methyl-aminomethan, aminopropandiole, sovak-base, 1-amino-2,3,4-butantriole.

Compounds of the Formula I of the present invention are preferred, wherein:
- A: is -meta-phenylene- or -C₅-C₆-heteroarylene-,
- X: is a 2- to 6-membered alkylene tether which is unsubstituted or singly or multiply substituted by substituents selected from the group comprising C₁-C₄-alkoxy, hydroxy, amino, -NH-C₁-C₄-alkyl, -N(C₁-C₄-alkyl)₂, or C₁-C₄-alkyl, which itself may be unsubstituted or singly or multiply substituted by C₁-C₄-alkoxy, hydroxy, amino, -NH-C₁-C₄-alkyl, or -N(C₁-C₄-alkyl)₂;
- Y: is -NR⁴- or -O-;
- R¹ and R²: are the same or different and are independently from each other selected from the group comprising hydrogen, hydroxy, halogen, nitro, cyano, -(CH₂)ₚ-NR⁵R⁶, -(CH₂)ₚ-NR⁵COR⁶, -(CH₂)ₚ-NR⁵S(O)₂R⁶, -(CH₂)ₚ-NR⁵CONR⁶R⁷, -(CH₂)ₚ-NR⁵COOR⁶, -(CH₂)ₚ-COR⁵, -(CH₂)ₚ-CR⁴(OH)-R⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-CO₂R⁵, -(CH₂)ₚ-CONR⁵R⁶, -(CH₂)ₚ-OR⁵, -CR⁵(OH)-R⁶, -O-(CH₂)ₚ-COR⁵, -O-(CH₂)ₚ-CR⁴(OH)-R⁵,
and moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)ₚ-phenyl, -(CH₂)ₚ-C₅-C₆-heteroaryl, wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl, and wherein 0 to 2 methylene groups of -C₁-C₈-alkyl may be replaced independently of each other by -O-, -S-, -C(=O)-, -S(O)₂- or -NR⁴-;
- R³: is selected from the group comprising halogen, nitro, cyano, -C₁₋C₆-alkylthio, amino, benzodioxolyl, -NR⁵R⁶, -S(O)₂(C₁-C₆-alkyl), -CONR⁵R⁶, -COR⁵, -COOR⁵, -NR⁵-(CH₂)ₚ-NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷,
and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)ₚ-phenyl, -(CH₂)ₚ-C₅-C₁₀-heteroaryl, -O-phenyl, -O-C₅-C₁₀-heteroaryl, -phenylene-(CH₂)ₚ-R⁵,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -C₃-C₈-cycloalkyl, -C₃-C₈-heterocycloalkyl, -(CH₂)ₚ-phenyl, -(CH₂)ₚ-C₅-C₆-heteroaryl, -phenylene-(CH₂)ₚ-R⁵, -NR⁵R⁶, -C₁-C₆-hydroxyalkyl, -C₁-C₆-haloalkyl, -C₁-C₆-haloalkoxy, -C₂-C₆-alkenyl, -C₂-C₆-alkynyl, -C₁-C₆-alkoxy-C₁-C₆-alkyl, -(CH₂)ₚ-NR⁵CONR⁶R⁷, -(CH₂)ₚ-S(O)₂R⁵, -(CH₂)ₚ-NR⁵S(O)₂R⁶, -(CH₂)ₚ-S(O)₂NR⁵R⁶, -(CH₂)ₚ-CONR⁵R⁶, -(CH₂)ₚ-NR⁵COR⁶, -(CH₂)ₚ-CR⁴(OH)-R⁵, -O-(CH₂)ₚ-CR⁴(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein phenylene, -C₃-C₈-cycloalkyl, -C₃-C₈-heterocycloalkyl, -(CH₂)ₚ-phenyl and -(CH₂)ₚ-C₅-C₁₀-heteroaryl are unsubstituted or singly or multiply substituted independently from each other with halogen, hydroxy, -C₁-C₆-alkyl, -C₁-C₆-alkoxy, -CF₃ and/or -OCF₃, and wherein 0 to 2 methylene groups of -C₁-C₈-alkyl may be replaced independently of each other by -O-, -S-, -C(=O)-, -S(O)₂- or -NR⁴-;
- R⁴: is hydrogen or -C₁-C₈-alkyl;
- R⁵, R⁶,:
- R⁷ and R⁸: are the same or different and are independently from each other selected from the group comprising hydrogen, benzodioxolyl and moieties, said moieties being selected from the group comprising -C₁-C₈-alkyl, -C₃-C₈-cycloalkyl, -C₃-C₈-heterocydoalkyl, phenyl, -C₅-C₁₀-heteroaryl,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -C₃-C₈-cycloalkyl, -C₃-C₈-heterocycloalkyl, -C₁-C₆-hydroxyalkyl, -C₁-C₆-haloalkyl, -C₁-C₆-haloalkoxy, -NR⁹R¹⁰, -NR⁹CONR¹⁰, -S(O)₂(C₁-C₆-alkyl), -S(O)₂-phenyl, -NH-S(O)₂(C₁-C₆-alkyl), -NH-S(O)₂-phenyl, -S(O)₂-NH-(C₁-C₆-alkyl), -S(O)₂-NH-phenyl, -C₁-C₆-alkanoyl, -CONR⁹R¹⁰, -COR⁹, -COOR⁹, -(CH₂)ₚ-phenyl, -(CH₂)ₚ-C₅-C₆-heteroaryl, -phenylene-(CH₂)ₚ-R⁹,
wherein phenylene, -C₃-C₈-cycloalkyl, -C₃-C₈-heterocycloalkyl, phenyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or -NR⁴-;
or
two members selected from the group comprising R⁵, R⁶, R⁷ and R⁸ form a 2- to 8-membered alkylene tether which may be unsubstituted or singly substituted by -(CH₂)ₚ-OH or -(CH₂)ₚ-NR⁹R¹⁰, and in which 0 to 2 methylene groups may be replaced by -O- or -NR⁴-;
- R⁹, R¹⁰: are independently from each other hydrogen or -C₁-C₈-alkyl;
- p: is from 0 - 6.

More preferred are compounds of the Formula I of the present invention
wherein:
- A: is -meta-phenylene-;
- X: is an unsubstituted 4- to 5-membered alkylene tether;
- Y: is -NR⁴- or -O-;
- R¹ and R²: are the same or different and are independently from each other selected from the group comprising hydrogen, hydroxy, halogen, nitro, cyano, -(CH₂)ₚ-NR⁵R⁶, -(CH₂)ₚ-NHCOR⁶, -(CH₂)ₚ-NHS(O)₂R⁶, -(CH₂)ₚ-NHCONR⁶R⁷, -(CH₂)ₚ-NHCOOR⁶, -(CH₂)ₚ-COR⁵, -(CH₂)ₚ-CH(OH)-R⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-CO₂R⁵, -(CH₂)ₚ-CONR⁵R⁶, -(CH₂)ₚ-OR⁵, -CR⁵(OH)-R⁶, -O-(CH₂)ₚ-COR⁵, -O-(CH₂)ₚ-CH(OH)-R⁵
and moieties being selected from the group comprising -C₁-C₈-alkyl, -(CH₂)ₚ-phenyl, -(CH₂)ₚ-C₅-C₆-heteroaryl,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl;
- R³: is selected from the group comprising halogen, cyano, amino, benzodioxolyl, -NR⁵R⁶, -CONR⁵R⁶, -COR 5, -NR⁵-(CH₂)ₚ₋NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷,
and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)ₚ-phenyl, -(CH₂)ₚ-C₅-C₁₀-heteroaryl,
wherein said moieties are unsubstituted or singly or multiply substituted independently from each other with hydroxy, halogen, -C₁-C₆-alkoxy, amino, cyano, -C₁-C₆-alkyl, -C₃-C₈-cycloalkyl, -C₃-C₈-heterocycloalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR R , -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein -C₃-C₈-cydoalkyl, -C₃-C₈-heterocydoalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or -NR⁴-;
- R⁴: is hydrogen or -C₁-C₈-alkyl;
- R⁵, R⁶,:
- R⁷ and R⁸: are the same or different and are independently from each other selected from the group comprising hydrogen, benzodioxolyl and moieties, said moieties being selected from the group comprising -C₁-C₈-alkyl, -C₃-C₈-cycloalkyl, -C₃-C₈-heterocycloalkyl, phenyl, -C₅-C₁₀-heteroaryl,
wherein said moieties are unsubstituted or singly or multiply substituted independently from each other with hydroxy, halogen, -C₁-C₆-alkoxy, amino, cyano, -C₁-C₈-alkyl, -C₃-C₈-cycloalkyl, -C₁-C₆-hydroxyalkyl, -C₁-C₆-haloalkyl, -C₁-C₆-haloalkoxy, -NR⁹R¹⁰, -NR⁹CONR¹⁰, -S(O)₂(C₁-C₆-alkyl), -S(O)₂-phenyl, -NH-S(O)₂(C₁-C₆-alkyl), -NH-S(O)₂-phenyl, -C₁-C₆-alkanoyl, -CONR⁹R¹⁰, -COR⁹, -COOR⁹, -(CH₂)ₚ-phenyl, -(CH₂)ₚ-C₅-C₆-heteroaryl,
wherein -C₃-C₈-cycloalkyl, phenyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or-NR⁴-;
or
two members selected from the group comprising R⁵, R⁶, R⁷ and R⁸ form a 3- to 7-membered alkylene tether which may be unsubstituted or singly substituted by -(CH₂)ₚ-OH or -(CH₂)ₚ-NR⁹R¹⁰, and in which 0 to 2 methylene groups may be replaced by -O- or -NR⁴-;
- R⁹, R¹⁰: are independently from each other hydrogen or -C₁-C₈-alkyl;
- p: is from 0 - 4.

Furthermore compounds of the Formula I of the present invention are preferred,
wherein:
R⁴ is hydrogen, methyl, or ethyl.

Furthermore compounds of the present invention are preferred, wherein
- R⁵, R⁶, R⁷ and R⁸: are the same or different and are independently from each other selected from the group comprising hydrogen and moieties, said moieties being selected from the group comprising -C₃-C₈₋cycloalkyl, -C₃-C₈-heterocycloalkyl, -C₁-C₈-alkyl, phenyl and -C₅-C₆-heteroaryl,
wherein said moieties are unsubstituted or singly or multiply substituted independently from each other with hydroxy, halogen, -C₁-C₆-alkoxy, amino, cyano, -C₁-C₆-alkyl, -C₁-C₆-hydroxyalkyl, -C₁-C₆-haloalkyl, -C₁-C₆-haloalkoxy, -NR⁹R¹⁰, -CONR⁹R¹⁰, -COR⁹, or
two members selected from the group comprising R⁵, R⁶, R⁷ and R⁸ form a 3- to 7-membered alkylene tether which may be unsubstituted or singly substituted by -(CH₂)ₚ-OH or -(CH₂)ₚ-NR⁹R¹⁰, and in which 0 to 2 methylene groups may be replaced by -O- or -NR⁴.

Furthermore compounds of the Formula I of the present invention are preferred,
wherein:
- R¹ and R²: are the same or different and are independently from each other selected from the group comprising hydrogen, -(CH₂)ₚ-NHCOR⁶, -(CH₂)ₚ-NHS(O)₂R⁶, -(CH₂)ₚ-NHCONR⁶R⁷, -(CH₂)ₚ-NHCOOR⁶, -(CH₂)ₚ-COR⁵, -(CH₂)ₚ-CH(OH)-R⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-CO₂R⁵, -(CH₂)ₚ-CONR⁵R⁶, -CR⁵(OH)-R⁶, -O-(CH₂)ₚ-COR⁵, -O-(CH₂)ₚ-CH(OH)-R⁵.

Herein compounds are more preferred, wherein:
- R¹ and R²: are the same or different and are independently from each other selected from the group comprising hydrogen, -(CH₂)ₚ-NHCOR⁶, -(CH₂)ₚ-NHS(O)₂R⁶, -(CH₂)ₚ-NHCONR⁶R⁷, -(CH₂)ₚ-NHCOOR⁶, -(CH₂)ₚ-COR⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-CONR⁵R⁶, -O-(CH₂)ₚ-COR⁵.

More preferred are compounds, wherein:
- R¹ and R²: are the same or different and are independently from each other selected from the group comprising hydrogen, -(CH₂)ₚ-NHCOR⁶, -(CH₂)ₚ-NHS(O)₂R⁶, -(CH₂)ₚ-NHCONR⁶R⁷.

Furthermore compounds of the Formula I of the present invention are preferred,
wherein:
- R¹ and R²: are the same or different and are independently from each other selected from the group comprising -C₁-C₈-alkyl, -(CH₂)ₚ-phenyl, -(CH₂)ₚ-C₅-C₆-heteroaryl,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl.

Furthermore compounds of the Formula I of the present invention are preferred,
wherein:
- R³: is selected from the group comprising halogen, cyano, amino, benzodioxolyl, -NR⁵R⁶, -CONR⁵R⁶, -COR⁵, -NR⁵-(CH₂)ₚ-NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷.

Herein compounds are more preferred, wherein:
- R³: is selected from the group comprising -NR⁵R⁶, -CONR⁵R⁶, -COR⁵, -NR⁵-(CH₂)ₚ-NR⁶CONR⁷R⁸,-NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷; or
- R³: is selected from the group comprising halogen, cyano, amino; or
- R³: is benzodioxolyl.

Furthermore compounds of the Formula I of the present invention are preferred,
wherein:
- R³: is selected from the group comprising -C₁-C₈-alkyl, -C₂-C₈-alkenyl, and -C₂-C₈-alkynyl, wherein said moieties are unsubstituted or singly or multiply substituted independently from each other with hydroxy, halogen, -C₁-C₆-alkoxy, amino, cyano, -C₁-C₆-alkyl, -C₃-C₈-cycloalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -(CH₂)ₚ-COOR⁵, -O-(CH₂)ₚ-R⁵.
wherein -C₃-C₈-cycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

Furthermore compounds are more preferred, wherein:
- R³: is selected from the group comprising -C₁-C₈-alkyl and -C₂-C₈-alkynyl, wherein said moieties are unsubstituted or singly or multiply substituted independently from each other with halogen, -C₁-C₆-alkoxy, C₁-C₆-alkyl, -(CH₂)ₚ-phenyl, -(CH₂)ₚ-C₅-C₆-heteroaryl, -NR⁵R⁶, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -(CH₂)ₚ-COR⁵,
wherein -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

Furthermore compounds of the Formula I of the present invention are preferred,
wherein:
- R³: is selected from the group comprising -C₃-C₈-cycloalkyl and -C₃₋C₈-heterocycloalkyl, and is unsubstituted or singly or multiply substituted independently from each other with hydroxy, halogen, -C₁-C₆-alkoxy, amino, cyano, -C₁-C₆-alkyl, -C₃-C₈-cydoalkyl, -C₃-C₈-heterocycloalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷, -(CH₂)p-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵ -(CH₂)ₚ-COOR⁵ -O-(CH₂)ₚ-R⁵,
wherein -C₃-C₈-cycloalkyl, -C₃-C₈-heterocycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

Furthermore compounds are more preferred, wherein:
- R³: is selected from the group comprising cyclopentyl, cyclohexyl, piperazinyl, piperidinyl, 1,4-diazepanyl and pyrrolidinyl, wherein said moieties are unsubstituted or singly or multiply substituted independently from each other with hydroxy, -C₁-C₆-alkoxy, cyano, -C₁-C₆-alkyl, -C₃-C₈-cycloalkyl, -C₃-C₈-heterocycloalkyl, -NR⁵R⁶, -C₁-C₆-hydroxyalkyl, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

Furthermore compounds are preferred, wherein:
- R³: is selected from the group comprising piperazinyl, piperidinyl, and pyrrolidinyl, wherein said moieties are unsubstituted or singly or multiply substituted independently from each other with -C₁-C₆-alkyl, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

Furthermore compounds are more preferred, wherein:
- R³: is piperazinyl and is unsubstituted or singly or multiply substituted independently from each other with -C₁-C₆-alkyl, -(CH₂)ₚ-S(O)₂R⁵, -(CH₂)ₚ-CONR⁵R⁶, -(CH₂)ₚ-CH(OH)-R⁵, -(CH₂)ₚ-COR⁵.

Furthermore compounds of the Formula I of the present invention are preferred, wherein:
- R³: is selected from the group comprising -(CH₂)ₚ-phenyl and -(CH₂)ₚ-C₅-C₁₀-heteroaryl,
wherein said moieties are unsubstituted or singly or multiply substituted independently from each other with hydroxy, halogen, -C₁-C₆-alkoxy, amino, cyano, -C₁-C₆-alkyl, -C₃-C₈-cycloalkyl, -C₃-C₈-heterocycloalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(P)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -(CH₂)ₚ-COOR , -O-(CH₂)ₚ-R⁵,
wherein -C₃-C₈-cydoalkyl, -C₃-C₈-heterocycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

Furthermore compounds are more preferred, wherein:
- R³: is selected from the group comprising -(CH₂)ₚ-phenyl and -(CH₂)ₚ-C₅-C₆-heteroaryl,
wherein said moieties are unsubstituted or singly or multiply substituted independently from each other with halogen, -C₁-C₆-alkoxy, cyano, -C₁-C₆-alkyl, -NR⁵R⁶, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

Furthermore compounds are preferred, wherein:
- R³: is selected from the group comprising phenyl and -C₅-C₆-heteroaryl,
wherein said moieties are unsubstituted or singly or multiply substituted independently from each other with halogen, -C₁-C₆-alkoxy, cyano, -C₁-C₆-alkyl, -NR⁵R⁶, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

Furthermore compounds are more preferred, wherein:
- R³: is phenyl and is unsubstituted or singly or multiply substituted independently from each other with halogen, -C₁-C₆-alkoxy, cyano, -C₁-C₆-alkyl, -NR⁵R⁶, -C₁-C₆-haloalkoxy, -(CH₂)ₚ-NHCONR⁶R⁷, -(CH₂)ₚ-NHS(O)₂R⁶, -(CH₂)ₚ-CONR⁵R⁶, -(CH₂)ₚ-NHCOR⁶, -(CH₂)ₚ-COR⁵, -O-(CH₂)ₚ-COR⁵.

Mostly preferred are the following compounds:
Compound 1.1:
   (RS)-1⁵ -Bromo-4-imino-4-thia-2,9-diaza-1(2.4)-pyrimidina-3(1,3)-benzenacyclononaphane-4-oxide
Compound 1.2:
   (RS)-1⁵-Iodo-4-imino-4-thia-2,9-diaza-1(2,4)-pyrimidina-3(1,3)-benzenacyclo-nonaphane-4-oxide
Compound 1.3:
   *N*-{4-[(RS)-4-Imino-4-oxo-4λ⁶-thia-2,9-diaza-1(2,4)-pyrimidina-3(1,3)-benzenacyclononaphan-1⁵-yl]phenyl}-*N*'-phenyl urea
Compound 1.4:
   *N*-{4-[(*RS*)-4-Imino-4-oxo-4λ⁶-thia-2,9-diaza-1(2,4)-pyrimidina-3(1,3)-benzenacyclononaphan-1⁵-yl]phenyl}-*N'*-[3-(trifluormethyl)phenyl]urea
Compound 1.5:
   *N*-{4-[(*R*S)-4-Imino-4-oxo-4λ⁶-thia-2,9-diaza-1(2,4)-pyrimidina-3(1,3)-benzenacyclononaphan-1⁵-yl]phenyl}-*N*'-(3-methylphenyl)urea
Compound 1.6:
   *N*-(3-Ethylphenyl)-*N*'-{4-[(*RS*)-4-imino-4-oxo-4λ⁶-thia-2,9-diaza-1 (2,4)-pyrimidina-3(1,3)-benzenacyclononaphan-1⁵-yl]phenyl}urea

The compounds of the present invention can be used in treating diseases of dysregulated vascular growth or diseases which are accompanied by dysregulated vascular growth. Especially the compounds effectively interfere with angiopoietin and therefore influence Tie2 signalling. Surprisingly the compounds block Tie2 signalling, wherein obviously Tie2 kinase activity is blocked with showing no or very low cell toxicity for cells other than endothelial cells at low concentrations, which is an important advantage over prior art substances. This effect will therefore allow prolonged treatment of patients with the compounds offering good tolerability and high anti-angiogenic efficacy, where persistent angiogenesis plays a pathologic role.

Therefore the compounds of the present invention can be applied for the treatment of diseases accompanied by neoangiogenesis. This holds principally for all solid tumours, e.g. mamma, colon, renal, lung and/or brain tumours and can be extended to a broad range of diseases, where pathologic angiogenesis is persistent. This applies for diseases with inflammatory association, diseases associated with edema of various forms and diseases associated with stromal proliferation and pathologic stromal reactions broadly. Particularly suited is the treatment for gynaecological diseases where inhibition of angiogenic, inflammatory and stromal processes with pathologic character can be achieved. At the same time the toxic side effects on normal proliferating tissue are low. The treatment is therefore an addition to the existing armament to treat diseases associated with neoangiogenesis.

The compounds of the present invention can be used in particular in therapy, and prevention of tumour growth and metastases especially in solid tumours of all indications and stages with or without pre-treatment if the tumour growth is accompanied with persistent angiogenesis. However it is not restricted to tumour therapy but is also of great value for the treatment of other diseases with dysregulated vascular growth. This includes retinopathy and other angiogenesis dependent diseases of the eye (e.g. cornea transplant rejection, age-related macular degeneration), rheumatoid arthritis, and other inflammatory diseases associated with angiogenesis like psoriasis, delayed type hypersensitivity, contact dermatitis, asthma, multiple sclerosis, restenosis, pulmonary hypertension, stroke and inflammatory diseases of the bowel, like Crohn's disease. Such diseases include coronary and peripheral artery disease. It can be applied for disease states like ascites, edema, like brain tumour associated edema, high altitude trauma, hypoxia induced cerebral edema, pulmonary edema and macular edema or edema following burns and trauma. Furthermore it is useful for chronic lung disease, adult respiratory distress syndrome. Also for bone resorption and for benign proliferating diseases like myoma, benign prostate hyperplasia and wound healing for the reduction of scar formation. It is therapeutically valuable for the treatment of diseases, where deposition of fibrin or extracellular matrix is an issue and stroma proliferation is accelerated (e.g. fibrosis, cirrhosis, carpal tunnel syndrome etc). In addition it can be used for the reduction of scar formation during regeneration of damaged nerves, permitting the reconnection of axons. Further uses are endometriosis, pre-eclampsia, postmenopausal bleeding and ovarian hyperstimulation.

A second aspect of the invention is a pharmaceutical composition which contains at least one compound of general Formula I, e.g. in form of a pharmaceutically-acceptable salt, or an in vivo hydrolysable ester of at least one compound of general Formula I, and one or more pharmaceutically-acceptable diluents or carriers. This composition is particularly suited for the treatment of diseases of dysregulated vascular growth or of diseases which are accompanied with dysregulated vascular growth as explained above.

For using the compounds of the present invention as pharmaceutical product the compounds or mixtures thereof are provided in a pharmaceutical composition, which beside the compounds of the present invention for enteral, oral or parenteral application contain suitable pharmaceutically acceptable organic or inorganic inert base material, e.g. purified water, gelatin, rubber arabicum, lactate, starch, magnesium stearate, talcum, vegetable oils, polyalkylenglycole, etc.

The pharmaceutical composition may be provided in a solid form, e.g. as tablets, dragèes, suppositories, capsules or in liquid form, e.g. as a solution, suspension or emulsion. The pharmaceutical composition may additionally contain auxiliary substances, e.g. conservators, stabilisers, wetting agents or emulgators, salts for adjusting the osmotic pressure or buffers.

For parenteral application (including intravenous, subcutaneous, intramuscular, intravascular or infusion) sterile injection solutions or suspensions are preferred, especially aqueous solutions of the compounds in polyhydroxyethoxy containing castor oil.

The pharmaceutical composition may further contain surface active agents, *e.g.* salts of gallenic acid, phosphorlipids of animal or vegetable origin, mixtures thereof and liposomes and parts thereof.

For oral application tablets, dragèes or capsules with talcum and/or carbonhydrogen carriers and -binders, *e.g.* lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 - 1.500 mg. A dose can be administered as unit dose or in part thereof and distributed over the day. Accordingly the optimum dosage may be determined by the practitioner who is treating any particular patient.

Another aspect of the present invention is the method of preparing the compounds according to the present invention.

The following table lists the abbreviations used in this paragraph, and in the Examples section. NMR peak forms are stated as they appear in the spectra, possible higher order effects have not been considered.

| **Abbreviation** | **Meaning** |
|---|---|
| br | broad |
| d | doublet |
| dd | doublet of doublet |
| DCM | dichloromethane |
| DIPEA | N,N-diisopropylethyl amine |
| DMF | N,N-dimethylformamide |
| DMSO | Dimethyl sulfoxide |
| eq | equivalent |
| ESI | electrospray ionisation |
| GP | general procedure |
| m | multiplet |
| me | centred multiplet |
| mp. | melting point |
| MS | mass spectrometry |
| NMR | nuclear magnetic resonance spectroscopy |
| POPd | Dihydrogen dichlorobis(di-tert-butyl phosphinito-κP)palladate(2); CombiPhos Catalysts, Inc. |
| q | quartet |
| s | singlet |
| t | triplet |
| TEA | triethylamine |
| THF | tetrahydrofuran |
| TLC | thin layer chromatography |

If the production of the compounds of general Formula I according to the invention is not described, the latter is carried out analogously to known methods.

The structural determination of the macrocyclic fragrances Muskon and Zibeton by Ruzicka ((a) Ruzicka, L. Helv. Chim. Acta 1926, 9, 715. (b) Ruzicka, L. Helv. Chim. Acta 1926, 9, 249) in 1926 marks the beginning of the chemistry of macrocyclic compounds.

In general, medium (8- to 11-membered rings, expressed as C₈-C₁₁ according to the definition above) and large (≥ 12-membered rings) ring systems are referred to as macrocyclic compounds of general Formula I of the present invention. The established processes for synthesis of macrocyclic compounds are partially based on ring enlargement reactions (Hesse, M. Ring Enlargement in Organic Chemistry, VCH, Weinheim, 1991), and more rarely on ring contractions (Hayashi, T. J. Org. Chem. 1984, 49, 2326).

The most frequently used method is the cyclisation of bifunctional acyclic precursors (Review articles on the Synthesis of Macrocyclic Compounds: (a) Roxburgh, C. J. Tetrahedron 1995, 51, 9767. (b) Meng, Q. Top. Curr. Chem. 1991, 161, 107. (c) Paterson, I. Tetrahedron 1985, 41, 3569. (d) Masamune, S. Angew. Chem. 1977, 89, 602. (e) Nicolaou, K. C. Tetrahedron 1977, 33, 683).

The reactions outlined below are performed in the presence of a suitable solvent, for example, simple ketones, such as acetone; alcohols, such as, e.g., ethanol or butanol; esters, such as, for example, ethyl acetate; aromatic solvents, such as, for example, toluene or benzene; halogenated or halogen-free hydrocarbons such as hexane, dichloromethane, dichloroethane, or chloroform; ethers such as diethyl ether, tetrahydrofurane, 1,4-dioxane, or anisol as well as polar aprotic solvents, such as acetonitrile, DMSO, DMF or N-methylpyrrolidone, or mixtures of these solvents, also with the addition of water.

Suitable reducing agents are, for example, TiCl₃ and SnCl₂.

Certain steps, such as the formation of the macrocycles of the Formula I from their acyclic precursors, may require the presence of a suitable acid, for example inorganic acids such as hydrogen chloride, hydrogen bromide, sulfuric acid or BBr₃; organic acids such as acetic acid, formic acid, or trifluoroacetic acid; metal salts such as TiCl₃, SnCl₂, Ln(OTf)₃, etc.

Therein a method of preparing a compound of the present invention according to the general Formula I: 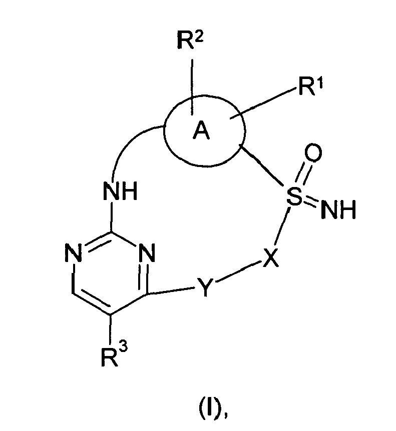 in which A, X, Y, R¹, R², and R³ have the meaning as given herein above, comprises the following general steps:
A) A disulfide of Formula **A** is transferred into a sulfoximine of Formula **B** 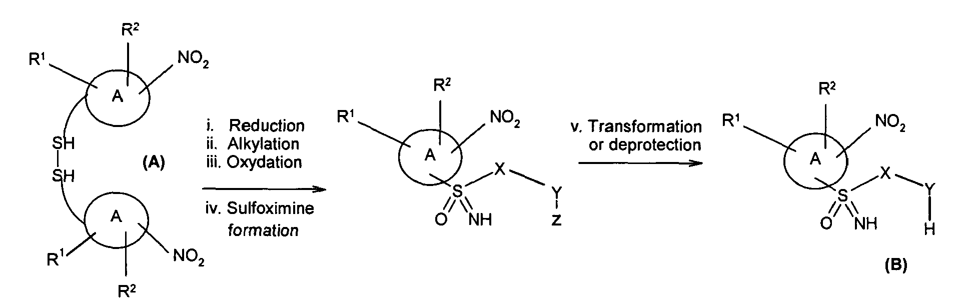 i. Reduction to the free thiol, ii. Alkylation with e.g. Z-Y-X-Halogen, iii. Oxidation to give the sulfoxide, iv. Transformation into the sulfoximine *e.g.* with NaN₃ /H₂SO₄ / CHCl₃; v. Transfomation or deprotection to give **B.**
   in which A, Y, R¹ and R² have the meaning as given herein above and Z is a protecting or activating group suitable e.g. to protect Y from oxidation or substitution, or to prepare Y for further reaction.
   Examples of these residues, which are well-known and merely illustrating, but not limiting the invention, may e.g. comprise a phthalimido moiety (resulting in compounds of the Formula I in which Y stands for -NH-) or a silyl ether such as *tert*-butyl dimethyl silyloxy or *tert-*butyl diphenylsilyloxy (resulting in compounds of the Formula I in which Y stands for -O-). Suitable deprotection reactions to transfer the intermediate Y-Z into free Y-H comprise e.g. the hydrazinolysis of a phthalimido moiety or the cleavage of a silyl ether by suitable reagents such as tetrabutyl ammonium fluoride. As an additional example, Y-Z could also refer to a suitable leaving group, such as a mesylate or a tosylate, which is then converted into a thioester by treatment with e.g. potassium thioacetate or potassium thiobenzoate. The free thiol (in which Y stands for -S-) can be generated by subsequent saponification with e.g. aqueous sodium hydroxide. Further suitable protecting groups and procedures for their introduction and cleavage are well known to those skilled in the art; particular reference is made to T. W. Greene and P. G. M. Wuts, Protective Groups in Organic Synthesis, John Wiley & Sons (1999).
B) The sulfoximine of Formula **B** is transferred into a sulfoximine of Formula **C** 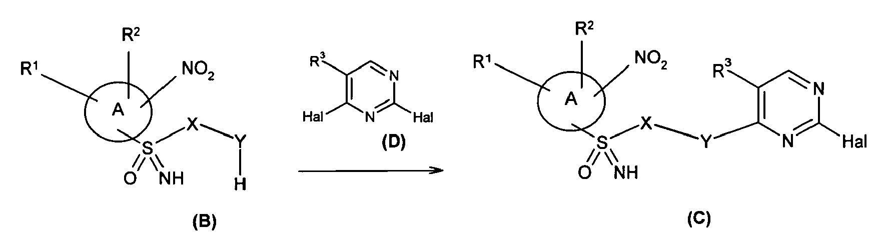 Coupling sulfoximine **B** with dihalopyrimidine **D,** e.g. under using Et₃N in acetonitrile.
   by coupling **B** with a dihalopyrimidine **D,** in which A, X, Y, R¹, R², and R³ have the meaning as given herein above, and wherein Hal is halogen. Suitable 2,4-dihalopyrimidine building blocks with various R³ are well known in the scientific literature and are partly commercially available. As outlined further below, R³ can also be further elaborated after completion of the macrocycle synthesis.
C) The sulfoximine of Formula C is transferred into a macrocyclic sulfoximine of Formula I 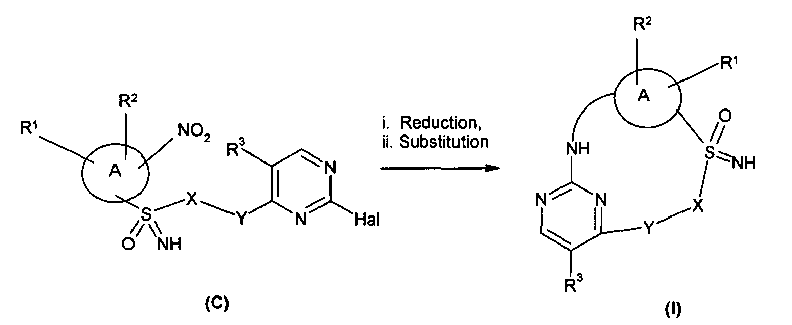 i. Reduction with *e.g.* TiCl₃ or SnCl₂, ii. Substitution *e.g.* HCl/dioxane in aq. acetonitrile.
in which A, X, Y, R¹, R² and R³ have the meaning as given herein above, by a sequence of a reduction of the nitro group in (C) with an appropriate agent (i.), followed by substitution of Hal on the pyrimidine-C-2 in (C) by the resulting amino group (ii.) e.g. by reacting with hydrochloric acid in an appropriate solvent at ambient or elevated temperature.

The sulfoximino-macrocycles of Formula I can be prepared, for example, starting from disulfides **A** which can be transferred into sulfoximines **B** according to literature procedures (*e.g.* i. Overman et al., Synthesis 1974 (1), 59; ii. Pasto et al., J. Am. Chem. Soc. 1994, 116, 8978; iii. Kim et al., Synthesis 2002 (17), 2484; iv. C. R. Johnson, J. Am. Chem. Soc. 1970, 92, 6594); for explicit protocols in this document, see the preparation of intermediates 1 to 4. Methods suitable for the subsequent transformation of sulfoximines **B** into the macrocycles of the Formula I are described in WO 2004/026881 A; such a sequence is also highlighted by the preparations of intermediates 5 and 8 and example compound 1.2 in this document.

The sulfoximino group (-S(=O)(=NH)-) can be generated e.g. from the corresponding sulfoxide either in free or substituted form (for a review article see e.g. M. Reggelin, C. Zur, Synthesis 2000, 1). Alternatively, the sulfoximine can be substituted on the NH group in a separate subsequent step. The resulting compounds featuring a substituted sulfoximino group -S(=O)(=NR^{x})- in which R^{x} is selected from the group comprising hydrogen, -COOR⁵, -C(=O)R⁵ , -COCHNR⁵R⁶, -S(O)₂R⁵, and -S(O)₂(CH₂)ₚ-Si(C₁-C₄₋alkyl)₃, in which p, R⁵ and R⁶ have the same meaning as given herein above.

Methods for the preparation of N-unsubstituted sulfoximines have been reported in the scientific literature, see e.g. C. R. Johnson, J. Am. Chem. Soc. 1970, 92, 6594; C. R. Johnson et al., J. Org. Chem, 1974, 39, 2458; H. Okamura, C. Bolm, Org. Lett. 2004, 6, 1305; the latter two methods also allow converting non-racemic sulfoxides, which are e.g. available by asymmetric oxidation of thioethers (see, for example, H. Kagan et al., J. Org. Chem. 1995, 60, 8086) into the corresponding sulfoximines without loss of stereochemical information.

The resulting sulfoximines can be subsequently reacted with suitable precursors of R^{x}, hereinafter referred to as R^{x}', to form the substituted sulfoximines -S(=O)(=NR^{x})-:

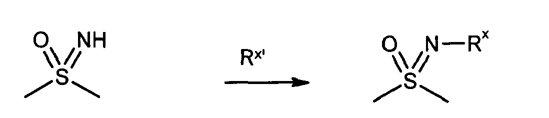

An example of R^{x}', which is merely illustrating but not limiting the invention is *e.g.* R^{x,} = Cl-C(=O)-OR⁵, resulting in compounds in which R^{x} is COOR⁵.

Alternatively, there are also methods known which directly lead to N-substituted sulfoximines -S(=O)(=NR^{x})-, see *e.g.* S. Cren et al., Tetrahedron Lett. 2002, 43, 2749, J. F. K. Mueller and P. Vogt, Tetrahedron Lett. 1998, 39, 4805, T. Bach and C. Korber, Tetrahedron Lett. 1998, 39, 5015.

Such substituted sulfoximino compounds may be useful intermediates in the preparation of compounds of the Formula I as they may prevent the NH group within the sulfoximine moiety from participating in undesired side reactions in the subsequent steps in the preparation of compounds of the Formula 1.

Furthermore, macrocycles of the Formula I according to the present invention (wherein R³ is shown as bromine/ iodine) may subsequently be further elaborated by modification of the R³ position to obtain other compounds according to the present invention in view of R³ position, for example by palladium catalysed coupling reactions such as Suzuki, Heck, or Sonogashira couplings, or further by amination methods if R³ is a halogen, preferably Br or I at the beginning of the reaction. Such aminations are well known to those skilled in the art and are widely described in the scientific literature; see e.g. J. C. Antilla, J. M. Baskin; T. E. Barder, S. L. Buchwald, J. Org. Chem. 2004, 69, 5578; T. A. Jensen, X. Liang, D. Tanner, N. Skjaerbaek, J. Org. Chem. 2004, 69, 4936; R. Varala, E. Ramu, M. M. Alam, S. R. Adapa, Synlett 2004, 10, 1737; C. Meyers, B. U. W. Maes, K. T. J. Loones, G. Bal, G. L. F. Lemière, R. A. Dommisse, J. Org. Chem. 2004, 69, 6010; EP 0103464 B1; or references cited therein. Similar introduction of an aryloxy or heteroaryloxy moiety is also possible, see *e.g.* E. J. Reinhard et al., J. Med. Chem. 2003, 46, 2152, and references cited therein.

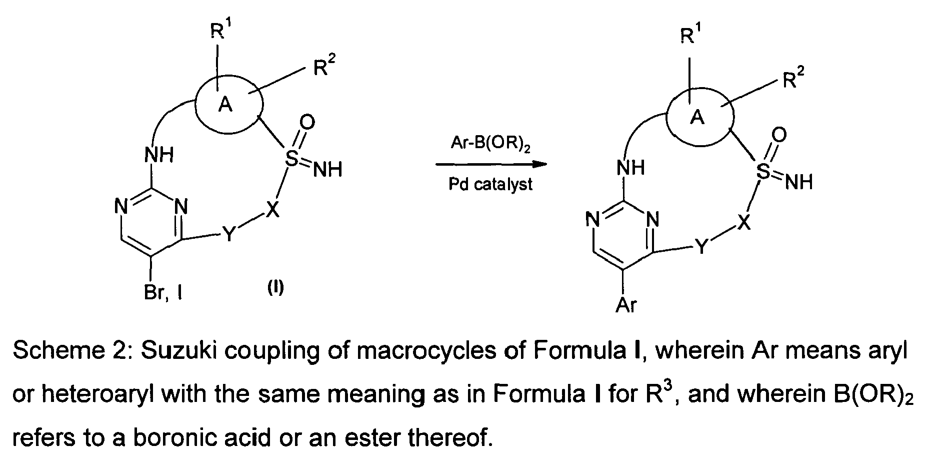

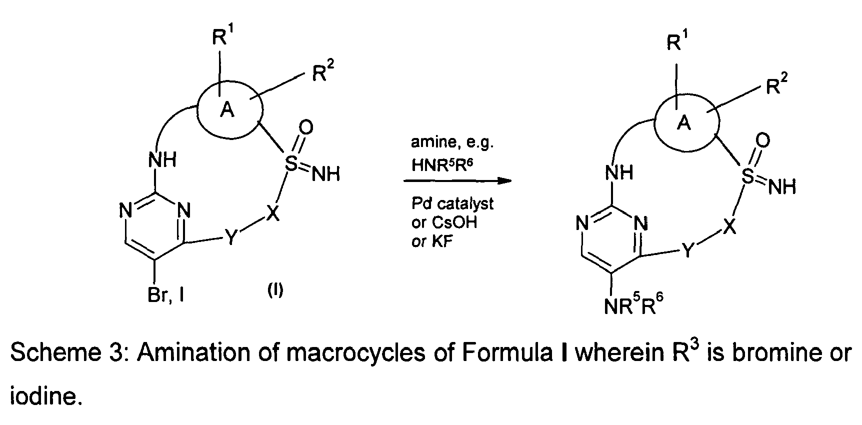

Appropriate coupling partners are either commercially available or can be prepared by simple standard functionalization procedures as shown in Scheme 4 well known to those skilled in the art.

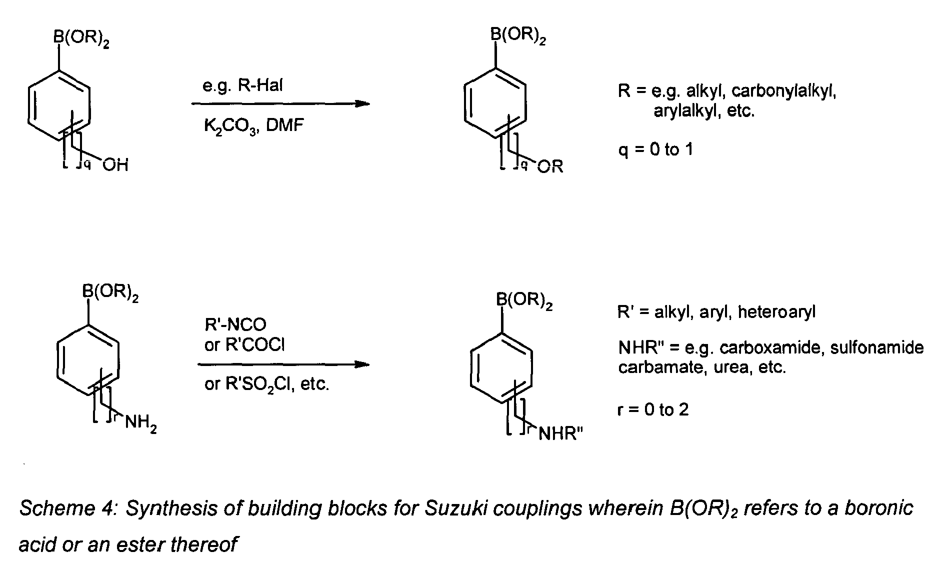

Additional methods suitable to prepare the compounds of the invention are well known and / or readily accessible to those skilled in the art and are described in the scientific literature, e.g. monographs such as R. Larock, Comprehensive Organic Transformations, VCH Publishers (1999), T. W. Greene and P. G. M. Wuts, Protective Groups in Organic Synthesis, John Wiley & Sons (1999), and L. Paquette, ed., Encyclopedia of Reagents for Organic Synthesis, John Wiley & Sons (1995), and in the sources cited in this document.

### Example compounds

The following examples illustrate the preparation of the compounds of the invention without limiting the scope of the claims.

### General procedures

### GP 1: Reaction of aminophenylboronic acid esters with isocyanates

A solution of the respective aminophenylboronic acid ester in DCM (5 mL per mmol boronic ester) was treated with the respective isocyanate (1.05 eq.), followed by TEA (1.1 eq.) at room temperature under an atmosphere of nitrogen. The resulting mixture was stirred overnight and then analysed by TLC. If the reaction did not reach completion after 20 h, additional reagents (isocyanate, 0.26 eq.; and TEA, 0.28 eq.) were supplemented and stirring was continued until the reaction was complete according to TLC. The mixture was evaporated and then subjected to column chromatography. In some cases, the intermediates obtained were still impure and used without further purification. Typically, the reactions were run on a 0.5 to 1 mmol scale.

### GP 2: Suzuki coupling

A solution of the respective macrocyclic halide in DMF (8 mL per mmol halide) was treated with the respective organoboron compound (1.25 eq.), K₂CO₃ (2.5 eq., either as a solid or as 2 M aqueous solution), and POPd (2.5 mol-%) at room temperature. The stirred resulting mixture was heated to 100 °C. The reaction was monitored by TLC, and additional portions of POPd (2.5 mol-%) and if consumed by then organoboron compound (1.25 eq.) were added. Stirring was continued for another 2 h, and addition of reagents, followed by 2 h stirring at 100 °C was repeated until the macrocyclic halide was completely consumed. After cooling to room temperature, water was added and the resulting suspension was stirred for 30 min. The crude product was isolated by vacuum filtration, dried *in vacuo,* and purified by column chromatography, followed by trituration with methanol, and / or preparative HPLC (e.g. YMC Pro C18RS 5µ, 150 x 20 mm, 0.2% NH₃ in water/acetonitrile) to give the pure coupling products.

Typically, the reactions were run on a 0.25 mmol scale.

### Preparation of intermediates

### Intermediate 1

### Preparation of 2-[4-(3-Nitro-phenylsulfanyl)-butyl]-isoindole-1 ,3-dione

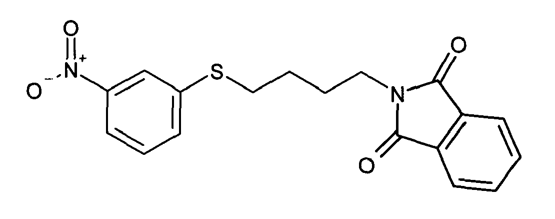

Triphenylphosphine (6.26 g, 23.75 mmol) was added to a solution of bis-(3-nitrophenyl)-disulfide (5.00 g, 16.21 mmol) in dioxane (65 mL) and water (16.3 mL), and the resulting mixture was stirred overnight at room temperature. The solvent was removed *in vacuo,* toluene was added, and the mixture was evaporated again. The residue was dissolved in ethanol (48. 5 mL) and treated with sodium hydroxide (850 mg, 21.25 mmol). To this mixture, 2-(4-bromobutyl)-isoindole-1,3-dione (8.52 g, 30.20 mmol) was added, followed by stirring overnight at room temperature. The reaction mixture was poured into water and was then extracted with ethyl acetate (3x). The combined organic layers were dried (Na₂SO₄), filtered and evaporated. The crude residue was purified by column chromatography (hexane/ethyl acetate 1:1) to give the desired product (7.10 g, 19.92 mmol, 61 % yield).
¹H-NMR (DMSO): 7.99 (m, 1H), 7.92 (m, 1H), 7.82 (s, 4H), 7.70 (m, 1H), 7.53 (m, 1 H), 3.59 (t, 2H), 3.13 (t, 2H), 1.78 (m, 2H), 1.62 (m, 2H).

### Intermediate 2

### Preparation of 2-[4-(3-Nitro-benzolsulfinyl)-butyl]-isoindole-1,3-dione

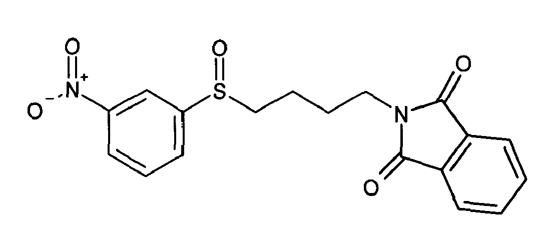

A solution of 2-[4-(3-nitro-phenylsulfanyl)-butyl]-isoindole-1,3-dione (3.90 g, 10.9 mmol) in acetonitrile (39 mL) was treated with anhydrous FeCl₃ (51 mg, 0.31 mmol) and periodic acid (2.65 g, 11.6 mmol). The resulting mixture was stirred for approx. 30 min at ambient temperature and then slowly poured into a solution of sodium thiosulfate (11.1 g) in ice water (75 mL). The mixture was left for another 30 min and was then extracted with DCM (2x). The combined organic layers were washed with dilute brine, dried (Na₂SO₄), filtered, and concentrated to give the title compound (4.14 g, 10.9 mmol, quantitative yield).
¹H-NMR (DMSO): 8.44 (m, 1 H), 8.32 (m, 1 H), 8.04 (m, 1 H), 7.82 (m, 5H), 3.54 (t, 2H), 3.15 (m, 1H), 2.89 (m, 1 H), 1.70 (m, 4H).
MS (ESI):[M + H]⁺ = 373.

### Intermediate 3

### Preparation of (RS)-S-(3-Nitrophenyl)-S-[4-(1,3-dioxo-2,3-dihydro-1H-isoindol-2-yl)butyl]sulfoximide

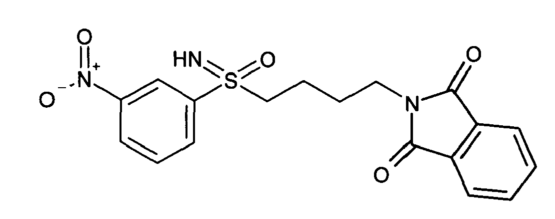

Concentrated sulphuric acid (2.8 mL) was added dropwise to a suspension of 2-[4-(3-nitro-benzolsulfinyl)-butyl]-isoindole-1,3-dione (2.07 g, 5.45 mmol) and sodium azide (795 mg, 12.23 mmol) in CHCl₃ (11.1 mL) under ice cooling. The resulting mixture was stirred at 45 °C for 25 h before being cooled to ambient temperature. Subsequently, ice-cooled diluted aqueous NaOH was added until an alkaline pH was maintained. The mixture was extracted with ethyl acetate (3x), and the combined organic layers were washed with brine, dried (Na₂S0₄), filtered and evaporated. The crude residue was purified by column chromatography (ethyl acetate) to give the desired product (1.30 g, 3.36 mmol, 62 % yield).
¹H-NMR (DMSO): 8.58 (m, 1 H), 8.46 (m, 1 H), 8.27 (m, 1 H), 7.85 (m, 5H), 4.62 (s, 1 H), 3.51 (t, 2H), 3.29 (m, 2H), 1.60 (m, 4H).

### Intermediate 4

### Preparation of (RS)-S-(4-Aminobutyl)-S-(3-nitrophenyl)sulfoximide

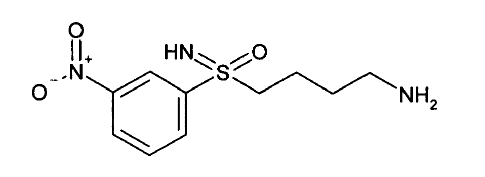

A solution of (RS)-S-(3-nitrophenyl)-S-[4-(1,3-dioxo-2,3-dihydro-1H-isoindol-2-yl)butyl]sulfoximide (1.29 g, 3.3 mmol) in ethanol (142 mL) was treated with hydrazine hydrate (3.4 mL) and heated under reflux for 6 h. Upon standing overnight at room temperature, a precipitate was formed which was isolated by vacuum filtration, and the filtrate was evaporated to give the title compound (920 mg, 3.3 mmol, quantitative yield).
¹H-NMR (DMSO): 8.60 (m, 1 H), 8.49 (m, 1 H), 8.30 (m, 1 H), 7.90 (m, 1 H), 4.59 (br, 1 H), 3.30 (m, 2H), 2.46 (m, 2H), 1.53 (m, 2H), 1.31 (m, 2H).

### Intermediate 5

### Preparation of (RS)-S-{4-[(5-Iodo-2-chloropyrimidin-4-yl)amino]butyl}-S-(3-nitrophenyl)sulfoximide

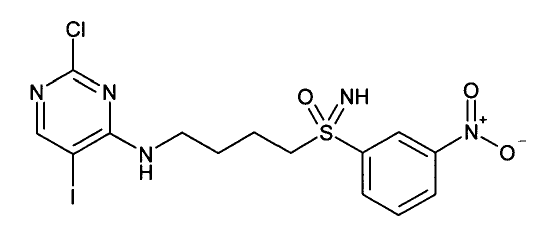

A solution of 2,4-dichloro-5-iodo-pyrimidine (586 mg, 2.14 mmol) in acetonitrile (2 mL) was added to a solution of (RS)-S-(4-aminobutyl)-S-(3-nitrophenyl)sulfoximide (550 mg, 2.14 mmol) in acetonitrile (8 mL) at room temperature. Triethylamine (0.30 mL) was added, and the resulting mixture was stirred for 23 h. The solvent was evaporated and the crude residue was purified by column chromatography (DCM / EtOH 95:5) to give the title compound (690 mg, 1.40 mmol, 65 % yield).
¹H-NMR (DMSO): 8.59 (m, 1 H), 8.49 (m, 1 H), 8.26 (m, 2H), 7.88 (m, 1 H), 7.32 (m, 1 H), 4.60 (s, 1 H), 3.30 (m, 4H), 1.54 (m, 4H).
MS (ESI):[M + H]⁺ = 496.

### Intermediate 6

### Preparation of (RS)-S-{4-[(5-Bromo-2-chloropyrimidin-4-yl)amino]butyl}-S-(3-nitrophenyl)sulfoximide

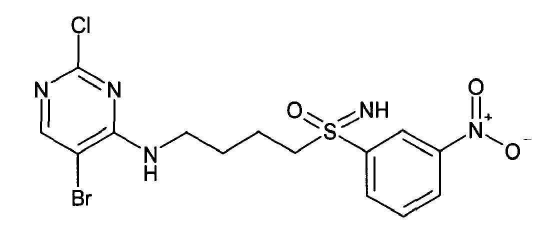

Triethylamine (0.27 mL) was added to a solution of (RS)-S-(4-aminobutyl)-S-(3-nitrophenyl)sulfoximide (200 mg, 0.78 mmol) and 5-bromo-2,4-dichloropyrimidine (177 mg, 0.78 mmol) in acetonitrile (3.5 mL), followed by stirring overnight at room temperature. The mixture was poured into brine and was then extracted with ethyl acetate (3x). The combined organic layers were dried (Na₂SO₄), filtered and evaporated. The crude residue was purified by column chromatography (ethyl acetate) to give the desired product (245 mg, 0.55 mmol, 70 % yield).
¹H-NMR (DMSO): 8.58 (m, 1H), 8.49 (m, 1H), 8.28 (m, 1H), 8.19 (s, 1H), 7.88 (m, 1 H), 7.70 (t, 1 H), 4.61 (s, 1 H), 3.30 (m, 4H), 1.53 (m, 4H).

### Intermediate 7

### Preparation of (RS)-S-{4-[(5-Bromo-2-chloropyrimidin-4-yl)amino]butyl) -N-(ethoxycarbonyl)-S-(3-nitrophenyl)sulfoximide

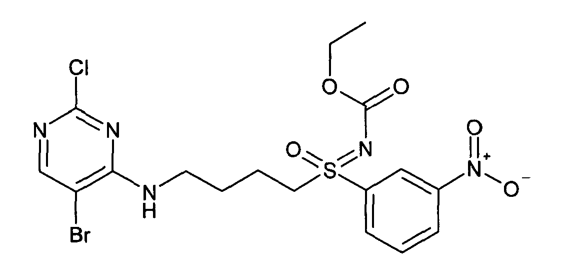

A solution of (RS)-S-{4-[(5-bromo-2-chloropyrimidin-4-yl)amino]butyl}-S-(3-nitrophenyl)sulfoximide (240 mg, 0.54 mmol) in pyridine (5 mL) was treated dropwise with ethyl chloroformiate (0.24 mL, 2.49 mmol) at ambient temperature. The resulting mixture was stirred for 5 h and was then poured into diluted brine. After extraction with ethyl acetate, the combined organic layers were dried (Na₂SO₄), filtered and evaporated to give the title compound (260 mg, 0.50 mmol, 93 % yield).
¹H-NMR (DMSO): 8.57 (m, 2H), 8.31 (m, 1H), 8.19 (s, 1H), 7.96 (m, 1H), 7.72 (t, 1 H), 3.91 (m, 2H), 3.75 (m, 2H), 3.30 (m, 2H), 1.61 (m, 4H), 1.07 (t, 3H).

### Intermediate 8

### Preparation of (RS)-S-(3-Aminophenyl)-S-{4-[(5-iodo-2-chloropyrimidin-4-yl)amino]butyl}sulfoximide

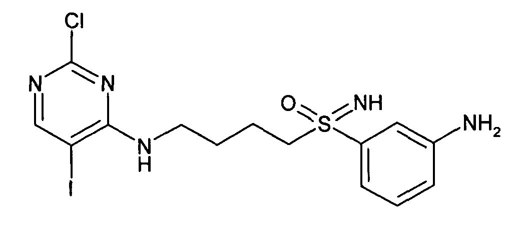

A 10 % solution of TiCl₃ in approx. 20 - 30% hydrochloric acid (12 mL, Aldrich) was added to a solution of (RS)-S-{4-[(5-iodo-2-chloropyrimidin-4-yl)amino]butyll-S-(3-nitrophenyl)sulfoximide (550 mg, 1.11 mmol) in THF (50 mL) at ambient temperature. After stirring for 30 min, another portion of 10 % solution of TiCl₃ in ~20 - 30% hydrochloric acid (1.5 mL) was added, and stirring at room temperature was continued for 30 min. Subsequently, the mixture was poured into aqueous 1 M NaOH containing crushed ice. The mixture was extracted with ethyl acetate (3x). The combined organic layers were dried (Na₂SO₄), filtered and evaporated. The crude residue was purified by column chromatography (DCM / EtOH 9:1) to give the desired product (300 mg, 0.65 mmol, 58 % yield).
MS (ESI):[M + H]⁺ = 466.

### Intermediate 9

### Preparation of (RS)-S-(3-Aminophenyl)-S-{4-[(5-bromo-2-chloropyrimidin-4-yl)amino]butyl}sulfoximide

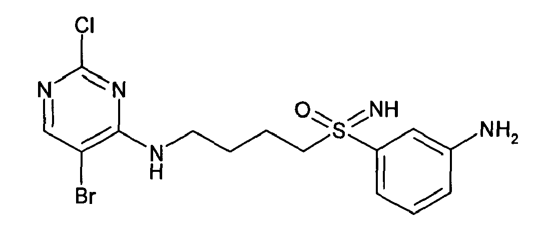

A 10 % solution of TiCl₃ in approx. 20 - 30% hydrochloric acid (1.7 mL, Aldrich) was added to a solution of (RS)-S-{4-[(5-bromo-2-chloropyrimidin-4-yl)amino]butyll-S-(3-nitrophenyl)sulfoximide (55 mg, 0.12 mmol) in THF (2.5 mL) at ambient temperature. The resulting mixture was stirred for 5 h at ambient temperature and was then poured into a mixture of aqueous 1 M NaOH and crushed ice. After saturation with NaCl, the mixture was extracted with ethyl acetate (3x). The combined organic layers were dried (Na₂S0₄), filtered and evaporated to give the title compound (48 mg, 0.11 mmol, 93 % yield).
¹H-NMR (DMSO): 8.21 (s, 1 H), 7.71 (t, 1 H), 7.15 (m, 1 H), 7.07 (m, 1 H), 6.93 (m, 1H), 6.74 (m, 1H). 5.53 (s, 2H), 3.88 (s, 1 H), 3.30 (m, 2H), 3.05 (m, 2H), 1.53 (m, 4H).

### Intermediate 10

### Preparation of (RS)-S-(3-Aminophenyl)-S-{4-[(5-bromo-2-chloropyrimidin-4-yl)amino]butyl}-N-(ethoxycarbonyl)sulfoximide

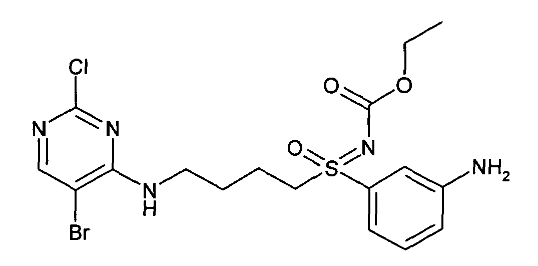

A 10 % solution of TiCl₃ in approx. 20 - 30% hydrochloric acid (6.7 mL, Aldrich) was added to a solution of (RS)-S-{4-[(5-bromo-2-chloropyrimidin-4-yl)amino]butyl}-N-(ethoxycarbonyl)-S-(3-nitrophenyl)sulfoximide (255 mg, 0.49 mmol) in THF (10 mL) at 0 °C under an atmosphere of nitrogen. The resulting mixture was stirred for 6 h at ambient temperature and was then poured into a mixture of aqueous 1 M NaOH and crushed ice. After saturation with NaCl, the mixture was extracted with ethyl acetate (3x). The combined organic layers were dried (Na₂SO₄), filtered and evaporated to give the title compound (179 mg, 0.36 mmol, 74 % yield).
¹H-NMR (DMSO): 8.21 (s, 1 H), 7.73 (t, 1 H), 7.22 (m, 1H), 7.03 (m, 1 H), 6.88 (m, 1 H), 6.82 (m, 1 H), 5.69 (s, 2H), 3.88 (m, 2H), 3.48 (m, 2H), 3.30 (m, 2H), 1.53 (m, 4H), 1.07 (t, 3H).

### Intermediate 11

### Preparation of Ethyl (RS)-N-[15-bromo-4-oxo-4 λ6-thia-2,9-diaza-1(2,4)-pyrimidina-3(1,3)-benzenacyclononaphan-4-ylidene]carbamate

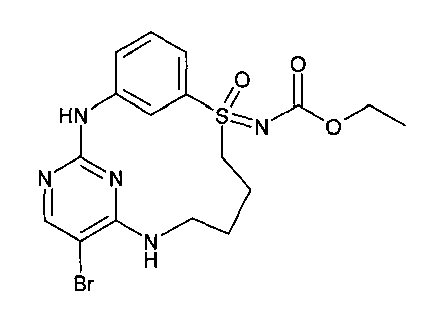

A solution of (RS)-S-(3-aminophenyl)-S-{4-[(5-bromo-2-chloropyrimidin-4-yl)amino]butyl}-N-(ethoxycarbonyl)sulfoximide (160 mg, 0.33 mmol) in acetonitrile (10 mL) was added to a mixture of acetonitrile / water / 4 M solution of HCI in 1,4-dioxane (45 mL / 5 mL / 0.5 mL) at a temperature of 80 °C over a period of 4 h by means of a syringe pump. After 24 h, all volatiles were removed *in vacuo* and sat. aq. NaHCO₃ solution (50 mL) and ethyl acetate (250 mL) were added. The organic layer was separated and the aqueous layer was extracted with ethyl acetate (4x). The combined organic layers were washed with brine, dried (Na₂SO₄), filtered and evaporated. The residue was triturated with *tert*.-butyl methyl ether to give the desired compound (135 mg, 0.30 mmol, 90 % yield).
¹H-NMR (DMSO): 9.69 (s, 1 H), 8.38 (m, 1 H), 8.04 (s, 1 H), 7.55 (m, 1 H), 7.47 (m, 1 H), 7.33 (m, 2H), 3.95 (q, 2H), 3.75 (m, 2H), 3.03 (m, 2H), 1.75 (m, 4H), 1.13 (t, 3H).
MS (ESI):[M + H]⁺ = 454.

### Intermediate 12:

### Preparation of N-phenyl-N'-[4-(4,4,5,5-tetramethyl-1,3,2-dioxaborolan-2-yl)phenyl]urea

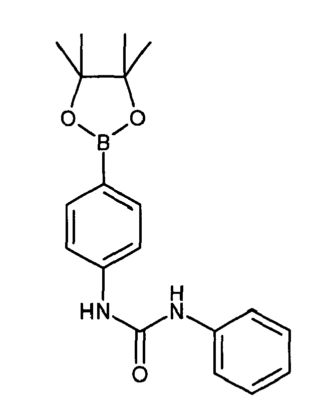

Intermediate 12 was prepared according to GP 1 from 4-(4,4,5,5-tetramethyl-1,3,2-dioxaborolan-2-yl)-aniline and phenyl isocyanate. Yield 70 %.
¹H-NMR (CDCl₃): 7.72 (d, 2H); 7.21 - 7.33 (m, 6H); 6.98 - 7.06 (m, 3H); 1.34 (s, 12H).
MS (ESI): [M+H]⁺ = 339.

### Intermediate 13:

### Preparation of N-[4-(4,4,5,5-tetramethyl-1,3,2-dioxaborolan-2-yl)phenyl]-N'-[3-(trifluoromethyl)phenyl]urea

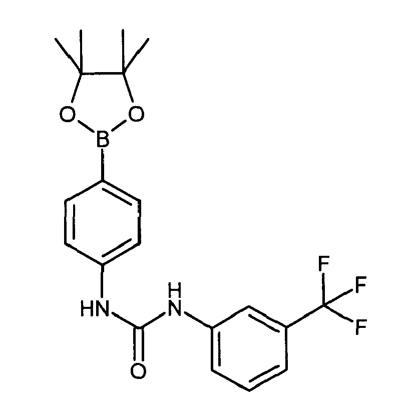

Intermediate 13 was prepared according to GP 1 from 4-(4,4,5,5-tetramethyl-1,3,2-dioxaborolan-2-yl)-aniline and 3-trifluoromethylphenyl isocyanate. Yield 89 %.
¹H-NMR (CDCl₃, 300 MHz): 7.74 (d, 2 H); 7.58-7.68 (m, 1 H): 7.16-7.55 (m, 7 H); 1.32 (s, 12 H).
MS (ESI): [M+H]⁺ = 407.

### Intermediate 14:

### Preparation of N-(3-methylphenyl)-N'-[4-(4,4,5,5-tetramethyl-1,3,2-dioxaborolan-2-yl)phenyl]urea

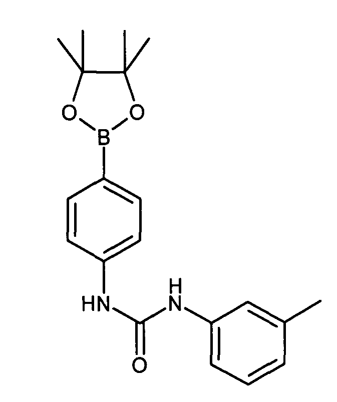

Intermediate 14 was prepared according to GP 1 from 4-(4,4,5,5-tetramethyl-1,3,2-dioxaborolan-2-yl)-aniline and 3-methylphenyl isocyanate. Yield 66 %.
¹H-NMR (DMSO): 8.74 (s, 1 H); 8.57 (s, 1 H); 7.55 (d, 2H); 7.44 (d, 2H); 7.26 (s, 1 H); 7.06 - 7.22 (m, 2H); 6.77 (d, 1 H); 2.25 (s, 3H); 1.27 (s, 12H).
MS (ESI): [M + H]⁺ = 353.

### Intermediate 15:

### Preparation of N-(3-ethylphenyl)-N'-[4-(4,4,5,5-tetramethyl-1,3,2-dioxaborolan-2-yl)phenyl]urea

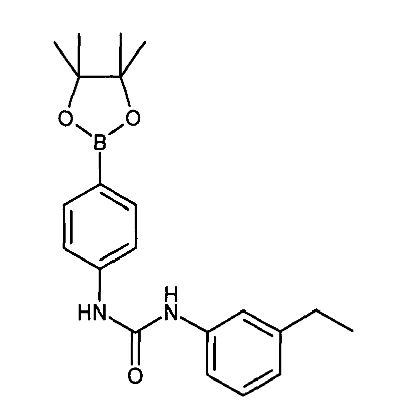

Intermediate 15 was prepared according to GP 1 from 4-(4,4,5,5-tetramethyl-1,3,2-dioxaborolan-2-yl)-aniline and 3-ethylphenyl isocyanate. Yield 37 %.
¹H-NMR (CDC13): 7.73 (d, 2H); 7.33 (d, 2H); 7.20 (t, 1 H); 7.14 (s br, 1 H); 7.06 - 7.13 (m, 2H); 6.99 (s br, 1 H); 6.93 (d, 1 H); 2.58 (q, 2H); 1.32 (s, 12H); 1.18 (t, 3H).
MS (ESI): [M + H]⁺ = 367.

### Preparation of example compounds

### Example 1.1

### Preparation of (RS)-1⁵-Bromo-4-imino-4-thia-2,9-diaza-1(2,4)-pyrimidina-3(1,3)-benzenacyclononaphane 4-oxide

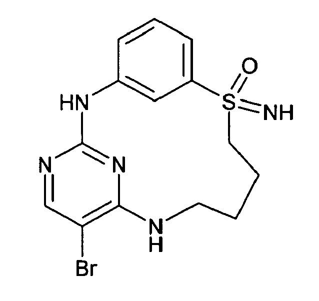

Ethyl (RS)-N-[1⁵-bromo-4-oxo-4 λ⁶-thia-2,9-diaza-1(2,4)-pyrimidina-3(1,3)-benzenacyclononaphan-4-ylidene]carbamate (81 mg; 0.18 mmol) was treated with a 0.125 M solution of sodium ethoxide in ethanol (4 mL) and heated to 70 °C. After 3 h and 4 h, portions of the 0.125 M solution of sodium ethoxide in ethanol (1 mL each) were added. Stirring at 70 °C was continued for another 5 h prior to cooling to room temperature, dilution with ethyl acetate, and the addition of brine. The resulting mixture was extracted with ethyl acetate (3x). The combined organic layers were dried (Na₂SO₄), filtered and evaporated. The residue was purified by column chromatography (DCM / EtOH 9:1) to give 35 mg (0.09 mmol, 52 % yield) of the title compound.
¹H-NMR (DMSO): 9.52 (s, 1H), 8.39 (m, 1H), 8.03 (s, 1H), 7.46 (m, 2H), 7.28 (m, 2H), 4.20 (s, 1H), 3.30 (m, 2H), 3.04 (m, 2H), 1.70 (m, 4H).
MS (ESI):[M + H]⁺ = 382.

The racemic title compound was separated into its enantiomers by preparative HPLC:

| | | |
|---|---|---|
| Column: | Chiralcel OJ 20µ | |
| Length x internal diameter: | 290 x 50.8 mm | |
| Eluents: | Hexane / Ethanol 70 : 30 | |
| Flow rate: | 80.0 mL / min | |
| Detector: | UV 280 nm | |
| Temperature: | room temperature | |
| Retention time [min]: | 43,7 : | Enantiomer 1 (Expl. 1a) |
| | 55,9 : | Enantiomer 2 (Expl. 1b) |

### Example 1.2

### Preparation of (RS)-1⁵-Iodo-4-imino-4-thia-2,9-diaza-1(2,4)-pyrimidina-3(1,3)-benzenacyclononaphane 4-oxide

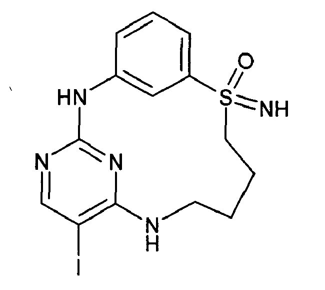

A solution of (RS)-S-(3-aminophenyl)-S-{4-[(5-iodo-2-chloropyrimidin-4-yl)amino]butyl}sulfoximide (295 mg, 0.63 mmol) in acetonitrile / water (10 mL /1mL) was added to a mixture of acetonitrile / water / 4 M HCI in 1,4-dioxane (90 mL /10 mL / 1 mL) at a temperature of 50 °C by means of a syringe pump over a period of 3 h. After 24 h, all volatiles were removed *in vacuo* and sat. aq. NaHCO₃ solution (50 mL) and ethyl acetate (250 mL) were added. The organic layer was separated and the aqueous layer was extracted with ethyl acetate (3x). The combined organic layers were washed with brine, dried (Na₂SO₄), filtered and evaporated. The crude residue was purified by column chromatography (DCM / EtOH 9:1) to give the title compound (135 mg, 0.32 mmol, 50 % yield).
¹H-NMR (DMSO): 9.51 (s, 1H), 8.39 (m, 1H), 8.12 (s, 1H), 7.45 (m, 2H), 7.23 (m, 1H), 6.95 (t, 1H), 4.19 (s, 1H), 3.30 (m, 2H), 3.04 (m, 2H), 1.69 (m, 4H).
MS (ESI):[M + H]⁺ = 430.

### Example 1.3

### Preparation of N-{4-[(RS)-4-Imino-4-oxo-4λ⁶-thia-2,9-diaza-1(2,4)-pyrimidina-3(1,3)-benzenacyclononaphan-1⁵-yl]phenyl}-N'-phenyl urea

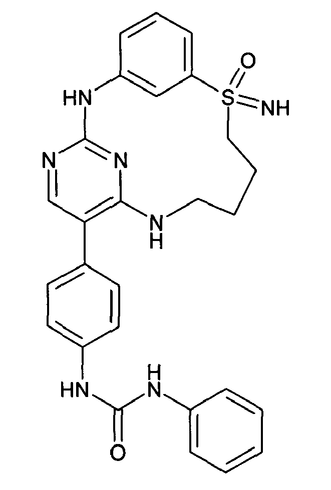

Example compound 1.3 was prepared according to GP 2 from (RS)-1⁵-Iodo-4-imino-4-thia-2,9-diaza-1(2,4)-pyrimidina-3(1,3)-benzenacyclononaphane 4-oxide and N-phenyl-N'-[4-(4,4,5,5-tetramethyl-1,3,2-dioxaborolan-2-yl)phenyl]urea. Yield 13%.
¹H-NMR (DMSO): 9.39 (s, 1H); 8.73 (s, 1H); 8.62 (s, 1H); 8.51 (s, 1H); 7.73 (s, 1H); 7.35 - 7.58 (m, 6H); 7.17 - 7.32 (m, 5H); 6.93 (t, 1H); 4.12 (s, 1H); 3.20 - 3.31 (m, 2H); 2.92 - 3.10 (m, 2H); 1.43 - 1.97 (m, 4H).
MS (ESI): [M + H]⁺ = 514.
mp. 267°C (decomposition).

### Example 1.4

### Preparation of N-{4-[(RS)-4-Imino-4-oxo-4λ⁶-thia-2,9-diaza-1(2,4)-pyrimidina-3(1,3)-benzenacyclononaphan-1⁶-yl]phenyl)-N-[3-(trifluormethyl)phenyl]urea

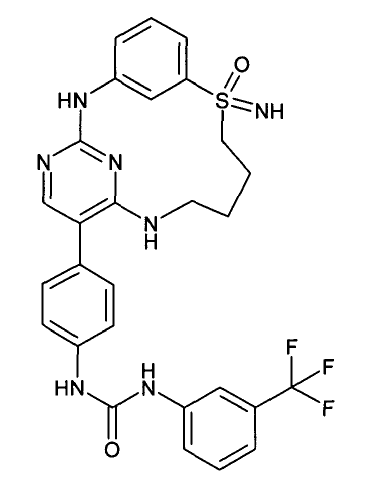

Example compound 1.4 was prepared according to GP 2 from (RS)-1⁵-lodo-4-imino-4-thia-2,9-diaza-1 (2,4)-pyrimidina-3(1,3)-benzenacyclononaphane 4-oxide and *N*-[4-(4,4,5,5-tetramethyl-1,3,2-dioxaborolan-2-yl)phenyl]-*N*'-[3-(trifluoromethyl)phenyl]urea. Yield 17%.
¹H-NMR (DMSO): 9.39 (s, 1H); 9.02 (s, 1H); 8.87 (s, 1H); 8.52 (s, 1H); 7.73 (s, 1H); 7.36-7.60 (m, 6H); 7.21-7.32 (m, 4H); 6.74 (t br, 1H); 4.12 (s, 1H); 3.21 - 3.32 (m, 2H); 2.93 - 3.08 (m, 2H); 1.43-1.98 (m, 4H).
MS (ESI): [M + H]⁺ = 582.
mp: 253°C (decomposition).

### Example 1.5

### Preparation of N-{4-[(RS)-4-lmino-4-oxo-4λ⁶-thia-2,9-diaza-1(2,4)-pyrimidina-3(1,3)-benzenacyclononaphan-1⁵-yl]phenyl}-N'-(3-methylphenyl)urea

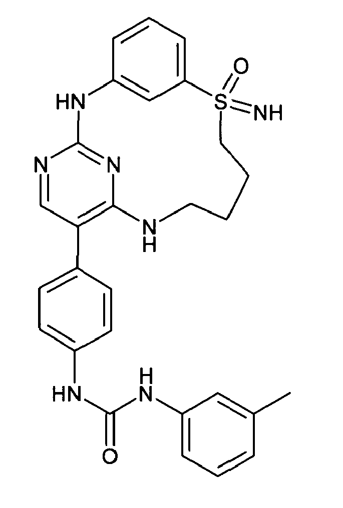

Example compound 1.5 was prepared according to GP 2 from (RS)-1⁵-lodo-4-imino-4-thia-2,9-diaza-1(2,4)-pyrimidina-3(1,3)-benzenacyclononaphane 4-oxide and *N*-(3-methylphenyl)-*N*'-[4-(4,4,5,5-tetramethyl-1,3,2-dioxaborolan-2-yl)phenyl]urea. Yield 7%.
¹H-NMR (DMSO): 9.42 (s, 1H); 8.73 (s, 1H); 8.58 (s, 1H); 8.50 (s, 1H); 7.73 (s, 1H); 7.46-7.55 (m, 2H); 7.33 - 7.44 (m, 2 H); 7.17 - 7.32 (m, 5 H); 7.12 (t, 1H); 6.70 - 6.80 (m, 2H); 4.16 (s, 1H); 3.20 - 3.29 (m, 2H); 2.93 - 3.06 (m, 2H); 2.23 (s, 3H); 1.68 - 1.93 (m, 2H); 1.46 - 1.67 (m, 2H).
MS (ESI): [M + H]⁺ = 528.
mp. 250°C (decomposition).

### Example 1.6

### Preparation of N-(3-Ethylphenyl)-N'-{4-[(RS)-4-imino-4-oxo-4λ⁶-thia-2,9-diaza-1(2,4)-pyrimidina-3(1,3)-benzenacyclononaphan-1⁵-yl]phenyl}urea

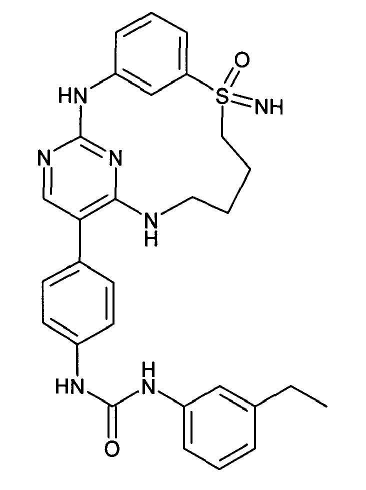

Example compound 1.6 was prepared according to GP 2 from (RS)-1⁵-Iodo-4-imino-4-thia-2,9-diaza-1(2,4)-pyrimidina-3(1,3)-benzenacyclononaphane 4-oxide and *N*-(3-ethylphenyl)-*N*'-[4-(4,4,5,5-tetramethyl-1,3,2-dioxaborolan-2-yl)phenyl]urea. Yield 16%.
¹H-NMR (DMSO): 9.42 (s, 1H); 8.72 (s, 1H); 8.59 (s, 1H); 8.50 (s, 1H); 7.74 (s, 1H); 7.48-7.56 (m, 2H); 7.33 - 7.45 (m, 2H); 7.30 (s, 1H); 7.19 - 7.27 (m, 4H); 7.15 (t, 1H); 6.72-6.82 (m, 2H); 4.16 (s, 1H); 3.20 - 3.28 (m, 2H); 2.92 - 3.08 (m, 2H); 2.56 (q, 2H); 1.70 - 1.94 (m, 2H); 1.44 - 1.69 (m, 2H); 1.13 (t, 3H).
MS (ESI): [M + H]⁺ = 542.
mp. 225°C (decomposition).

The following example compounds may be obtained using the methods described before or by standard procedures known to those skilled on the art:

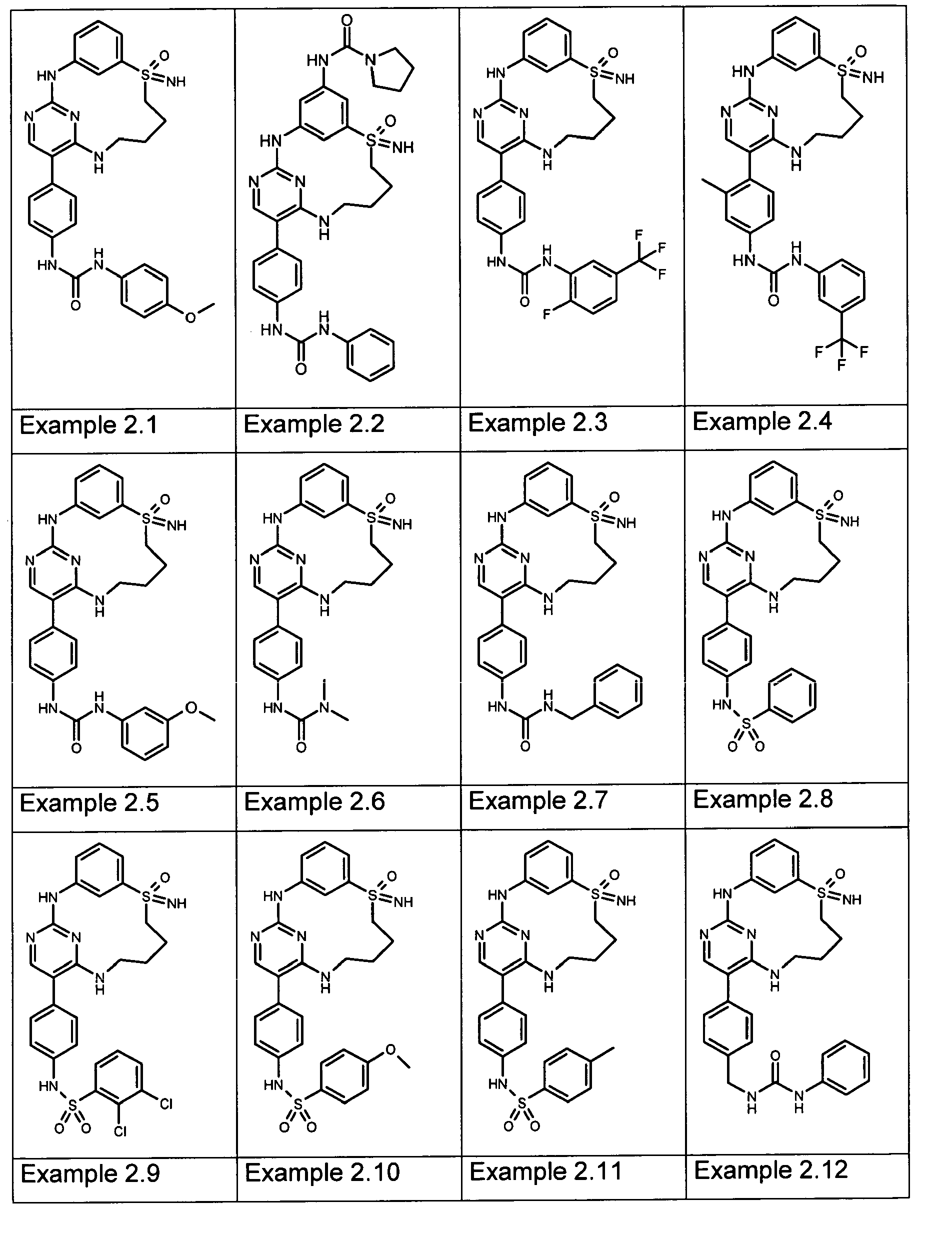

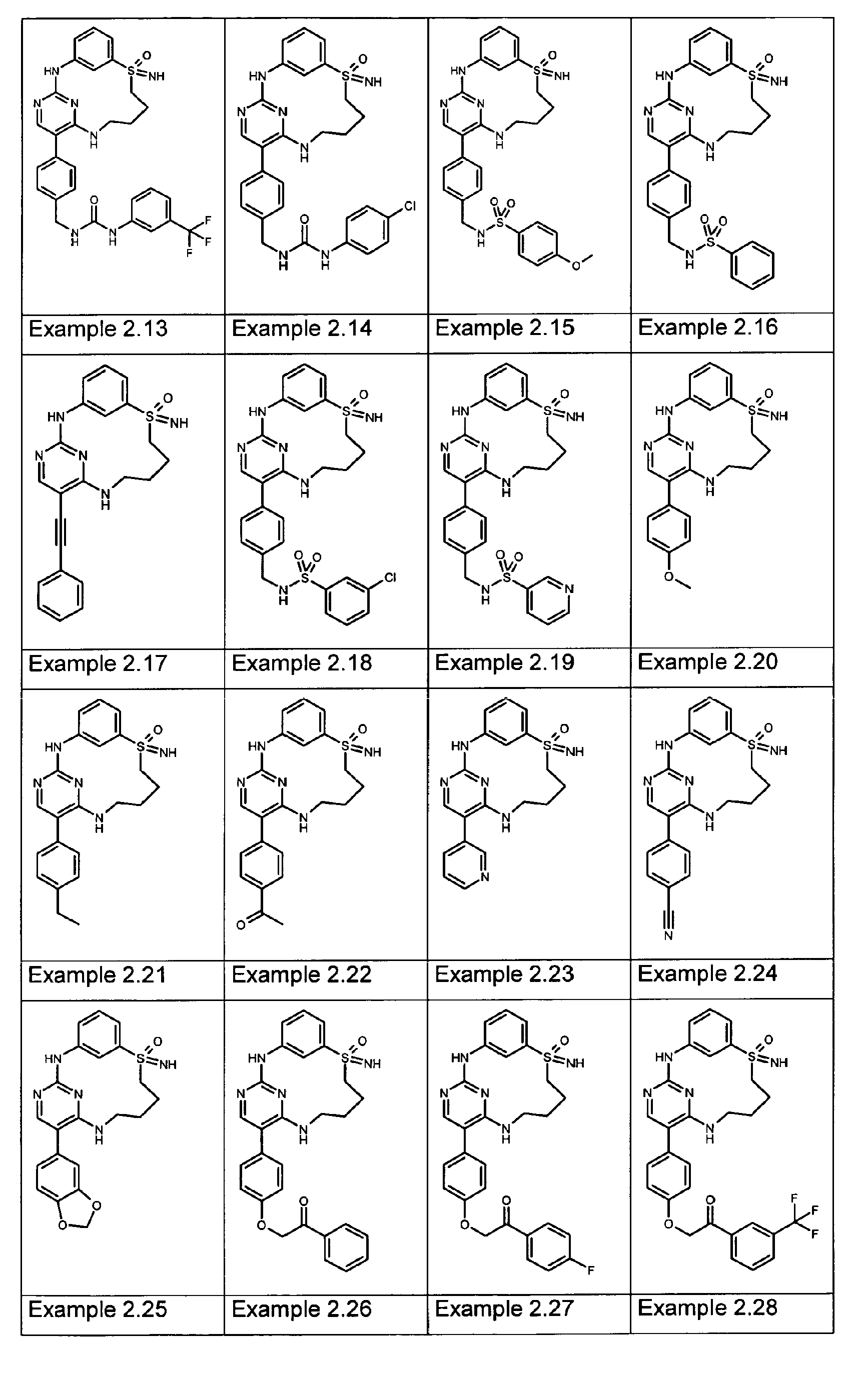

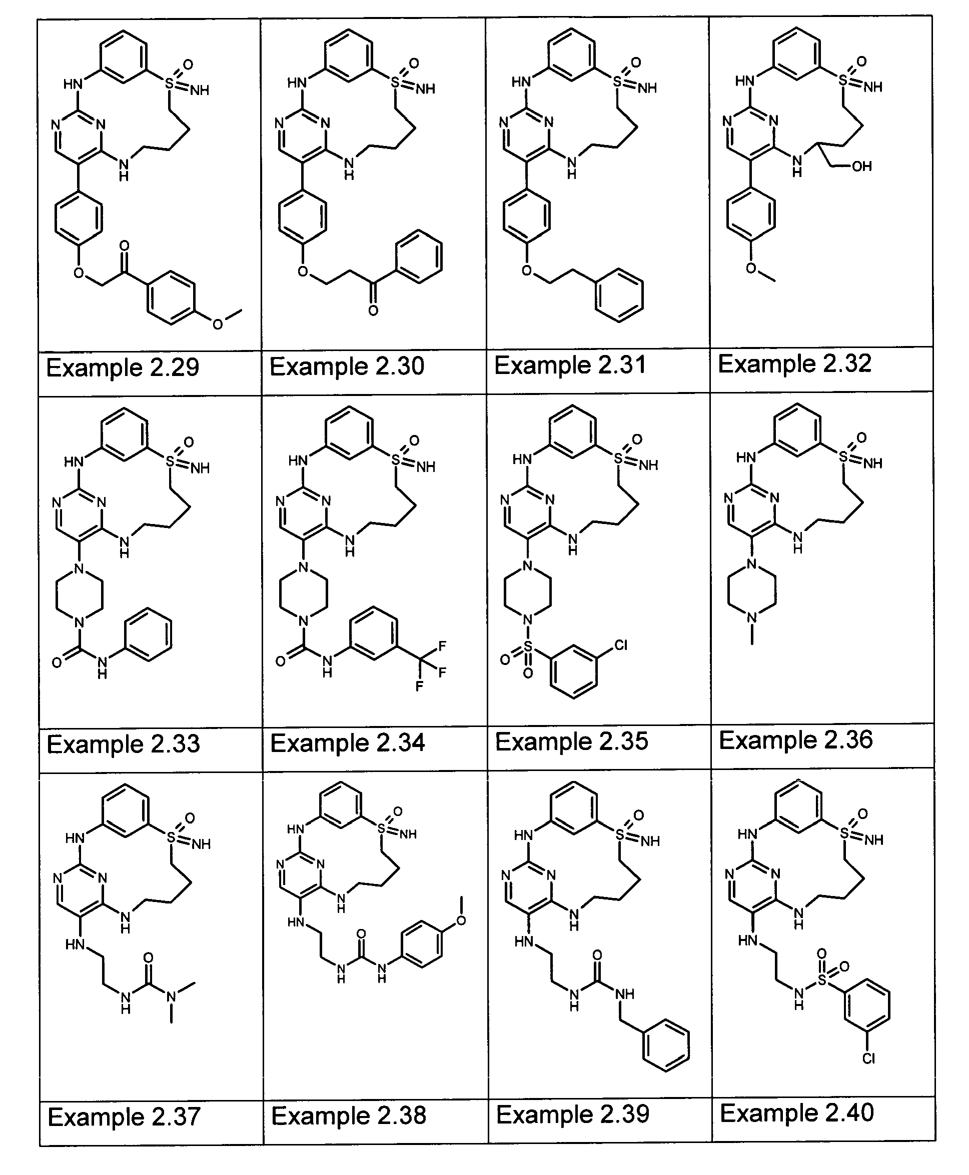

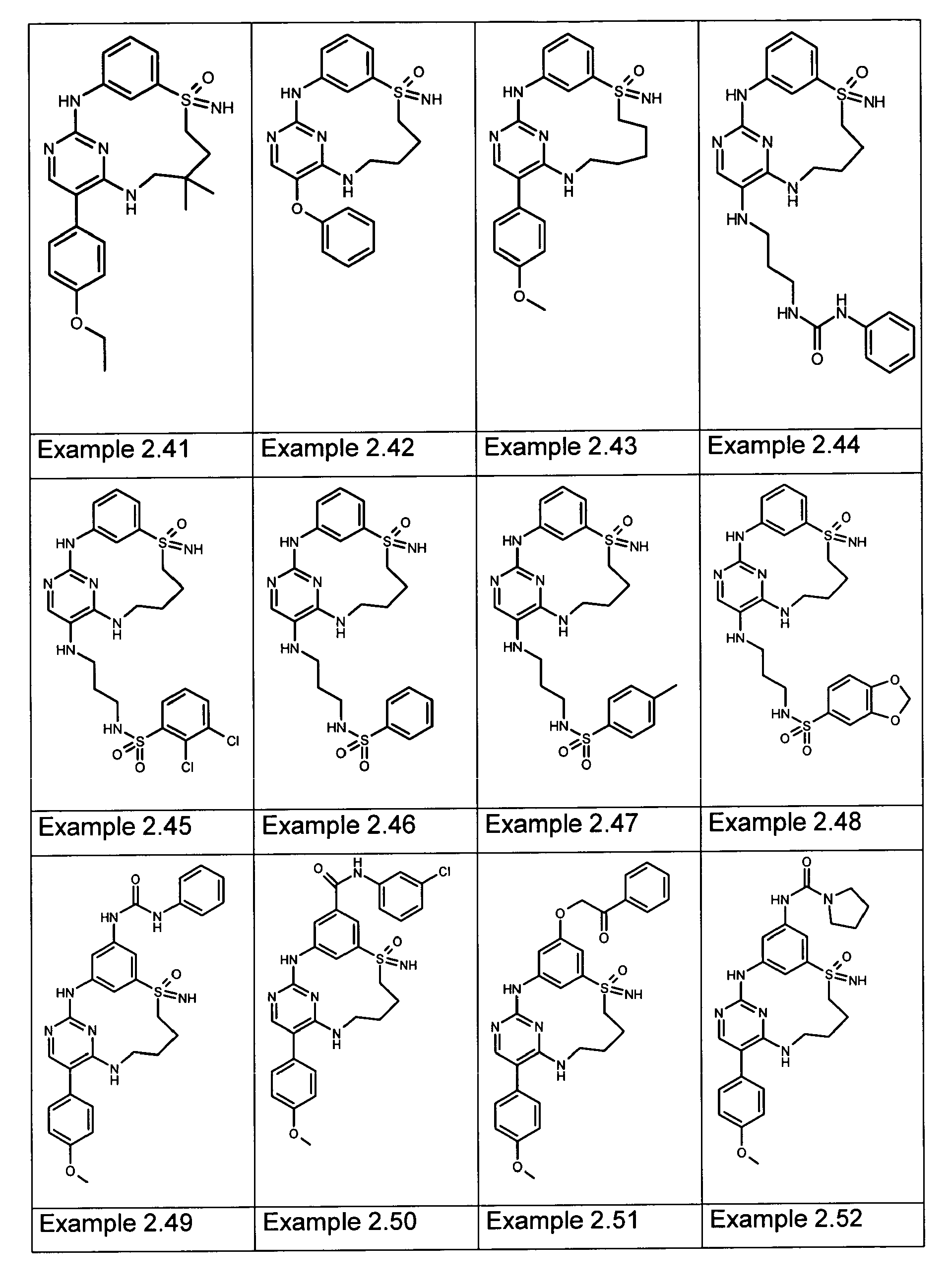

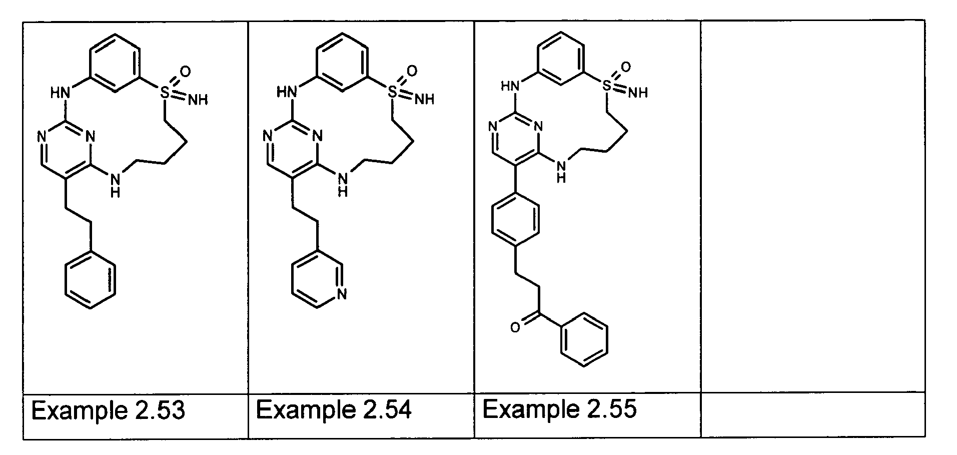

### Biological experiment 1: ELISA method

To prove the high potency activity as inhibitors of Tie2 kinase and Tie2 autophosphorylation the following ELISA-method was established and used.

Herein CHO cell-cultures, which are stably transfected by known techniques with Tie2 using DHFR deficiency as selection marker, are stimulated by angiopoietin-2. The specific autophosphorylation of Tie2 receptors is quantified with a sandwich-ELISA using anti-Tie2 antibodies for catch and anti-phosphotyrosine antibodies coupled to HRP as detection.

Materials:
96well tissue culture plate, sterile, Greiner
96well FluoroNunc plate MaxiSorp Surface C, Nunc
96well plate polypropylene for compound dilution in DMSO CHO Tie2/DHFR (transfected cells)
PBS-; PBS++, DMSO
MEM alpha Medium with Glutamax-I without Ribonucleosides and Deoxyribonucleosides (Gibco #32561-029) with 10% FCS after dialysis! and 1% PenStrep
Lysis buffer: 1 Tablet "Complete" protease inhibitor
   1 cap Vanadate (1 mL > 40 mg/mL; working solution 2 mM) ad 50 mL with Duschl-Puffer
   pH 7.6
Anti-TIE-II antibody 1 : 425 in Coating Buffer pH 9.6
   Stock solution: 1.275 mg/mL > working.: 3 µg/mL
PBST:2 bottles PBS(10x) + 10ml Tween, fill up with VE-water
RotiBlock 1 : 10 in VE-water
Anti-Phosphotyrosine HRP-Conjugated 1 : 10000 in 3% TopBlock 3% TopBlock in PBST
BM Chemiluminescence ELISA Substrate (POD) solution B 1 : 100 solution A
SF9 cell culture medium
Ang2-Fc in SF9 cell culture medium

### Cell experiment:

Dispense 5 x 10⁴ cells / well / 98 µL in 96well tissue culture plate Incubate at 37 °C / 5% CO₂
After 24 h add compounds according to desired concentrations
Add also to control and stimulated values without compounds 2 µL DMSO
And mix for a few min at room temperature
Add 100 µL Ang2-Fc to all wells except control, which receives insect medium
Incubate 20 min at 37 °C.
Wash 3x with PBS++
Add 100 µl Lysis buffer / well and shake a couple of min at room temperature
Store lysates at 20 °C before utilizing for the ELISA

### Performance of sandwich-ELISA

Coat 96well FluoroNunc Plate MaxiSorp Surface C with anti-Tie2 Mab 1 : 425 in Coating buffer pH 9.6; 100 µL /well overnight at 4 °C
Wash 2x with PBST
Blcock plates with 250 µL / well RotiBlock 1 : 10 in VE-water
Incubate for 2 h at room temperature or overnight at 4 °C shaking
Wash 2x in PBST
Add thawed lysates to wells and incubate overnight shaking at 4 °C Wash 2x with PBST
Add 100 µL/well anti-Phosphotyrosine HRP-Conjugated 1 : 10000 in 3% TopBlock (3% TopBlock in PBST) and incubate overnight under shaking
Wash 6x with PBST
Add 100 µL / well BM Chemiluminescence ELISA Substrate (POD) solutions 1 und 2 (1 : 100)
Determine luminescence with the LumiCount.

### Biological experiment 2: Tie-2-Kinase HTRF-Assay

To prove the effectiveness of the compound according to the present invention a Tie-2-Kinase HTRF-Assay was established.

Tie-2 phosphorylates tyrosine residues of the artificial substrate polyGAT (biotinylated polyGluAlaTyr). Detection of phosphorylated product is achieved specifically by a trimeric detection complex consisting of the phosphorylated substrate, streptavidin-XLent (SA-XLent) which binds to biotin, and Europium Cryptate-labeled anti-phosphotyrosine antibody PT66 which binds to phosphorylated tyrosine. Excitation of Europium fluorescence with 337 nm light results in emission of long-lived light with 620 nm. In case a trimeric detection complex has formed, part of the energy will be transferred to the SA-XLent fluorophore that itself then emits long-lived light of 665 nm (FRET: fluorescence resonance energy transfer). Unphosphorylated substrate does not give rise to light emission at 665nm, because no FRET-competent trimeric detection complex can be formed. Measurement is performed in a Packard Discovery or BMG Rubystar instrument. A-counts (emission at 665 nm) will be divided by B-counts (emission at 620 nm) and multiplicated with a factor of 10000. The resulting numbers are called the "well ratio" of the sample.

### Material:

Enzyme: Tie-2-Kinase, in house, aliquots (12 x 10 mL) stored at -80 °C
Substrate: PolyGAT labeled with Biotin (1000 µg / mL); CIS Bio ; # 61GATBLB; aliquots stored at -20 °C
ATP: Amersham Pharmacia Biotech Inc. # 27-2056-01; 100 mM; stored at -20 °C
Antibody: PT66-Eu Cryptate ; CIS Bio ; # 61T66KLB ; 30µg/mL; aliquots stored at -20 °C
SA-XLent ; CIS Bio; # 611SAXLB ; 1000 µg/mL; aliquots stored at -80 °C
Microplates : 384 Well black, SV, Greiner, # 784076

### Solutions:

Assay buffer:
50 mM HEPES (pH 7.0), 25 mM MgCl₂, 5 mM MnCl₂, 1 mM DTT, 0.5 mM Na₃VO₄, 0.01% (v/v) NP40, 1x Complete EDTA free

### Enzyme working solution:

Tie-2 stock solution is diluted 1:250 in assay buffer

### Substrate working solution:

PolyGAT (1000 µg/mL; 36.23 µM) is diluted 1:90.6 to 400 nM or 77.3 ng/well, ATP (100 mM) is diluted 1 : 5000 to 20.0 µM. Both dilutions in assay buffer. Final assay concentrations: poly-GAT: 200 nM or 5.25 µg/mL, ATP: 10 µM (1 x Km each).

Detection solution: 50 mM HEPES (pH 7.0), BSA 0.2%, 0.6 M KF, 200 mM EDTA, PT66-Europium Cryptate 2.5 ng/well, SA-XLent Cis Bio 90 ng/well.

### Assay steps

All steps at 20 °C
1. 0.75 µL of compound solution in 30 % (v/v) DMSO
2. add 7 µL of substrate working solution
3. add 7 µL of enzyme working solution
4. incubate 75 min (reaction volume: 14.75 µL)
5. add 8 µL of detection solution
6. incubate 180 min or over night at 4 °C (total volume: 22.75 µL)
7. measure HTRF in Packard Discovery or BMG Rubystar instrument (delay 50 µs, integrated time 400 µs)

Final concentrations (in 14.75 µL reaction volume):
Enzyme: unknown
polyGAT (1 x Km): 200 nM (77.3 ng)
ATP (1 x Km): 10 µM
DMSO: 1.5 % (v/v)
Buffer conditions: 50 mM HEPES (pH 7.0), 25 mM MgCl₂, 5 mM MnCl₂, 1 mM DTT, 0.5 mM NaV04, 0.01 % (v/v) NP40, 1 x Complete

### Controls:

C₀: uninhibited reaction (DMSO only)
Cᵢ: inhibited reaction with 20 µM Staurosporine

### Biological experiment 3: Proliferation test

To examine cell toxicity a cell proliferation test were established.

With the cell proliferation test different tumour cell lines (e.g. Du 145) can be examined. The cells were dispensed in RPMI 1640 culture medium, supplied with 10 % (v/v) fetal calf serum plus 1 % (v/v) Penicillin/Streptomycin solution at a cell density of 2.000 cell/100µL medium/per well (96well plate). After three hours the cells were washed with PBS (containing calcium and magnesium). 100 µl of culture medium above with 0.1 % (v/v) fetal calf serum was added and cultured at 37°C and 5% CO₂-atmosphere. Next day compounds of the present invention diluted in DMSO for appropriate concentrations were added and further 100 µL culture medium 0.5 % (v/v) fetal calf serum. After 5 days cell culturing at 37°C and 5% CO₂-atmosphere cells were washed with PBS. 20 µL of glutaraldehyde solution (11 % (v/v)) is added and the cells were slightly shaken at room temperature for 15 min. After that the cell were washed 3 times and dried in the air. 100 µL of crystal violet solution (0.1 % at pH 3.5) were added and the cells were shaken for 30 min. The cells were washed with tap water and air-dried. The colour is dissolved with 100 µL of acetic acid (10 % (v/v)) under strong shaking for 5 min. The absorption was measured at 595 nm wavelength.

The compounds presented in this application have high potency activity as inhibitors of Tie2 kinase and/or Tie2 autophosphorylation as measured with the ELISA-method. The IC50 values are below 1 µM. At the same time the toxicity of the compounds is substantially lower which is different to other compounds in this structure class. Typically, the IC50 values determined in the DU 145 cytotoxicity assay are substantially higher as those determined in the Tie2 kinase or Tie2 autophosphorylation assay.

Certain compounds of the invention have been found be highly potent inhibitors of Tie2. More specifically, example compounds 1.3 to 1.6 throughout inhibit Tie2 with an IC50 of 1 µM or less either in the Tie2 kinase assay or in the Tie2 autophosphorylation ELISA test. While featuring high inhibitory potency against Tie2 kinase activity, certain compounds of the invention have been found to be particularly weakly cytotoxic or non-cytotoxic. More specifically, selected example compounds 1.3 to 1.6 showed IC50 values in the cytotoxicity assay using the cell line DU 145 which are at least five times higher as compared to those determined in the Tie2 kinase or Tie2 autophosphorylation assay.

The compounds of the present invention are therefore preferentially active as antiangiogenesis inhibitors and not as cytostatic or cytotoxic agents that affect tumour cells and other proliferating tissue cells directly.

## Claims

1. A compound of the general Formula I: 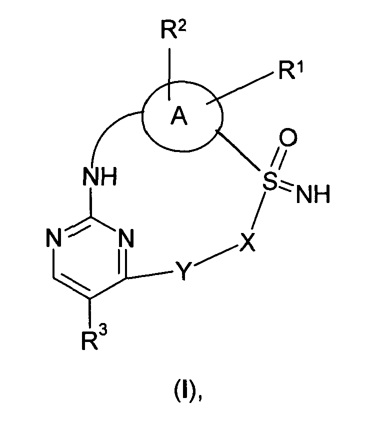 in which
A is -phenylene- or -C₅-C₆-heteroarylene-;
X is a 2- to 6-membered alkylene tether which is unsubstituted or singly or multiply substituted by substituents selected from the group comprising halogen, C₁-C₄-alkoxy, hydroxy, amino, cyano, carboxy, -NH-C₁-C₄-alkyl, -N(C₁-C₄-alkyl)₂, and C₁-C₄-alkyl, which itself may be unsubstituted or singly or multiply substituted by halogen, C₁-C₄-alkoxy, hydroxy, amino, cyano, carboxy, -NH-C₁-C₄-alkyl, or -N(C₁-C₄-alkyl)₂;
Y is -NR⁴-, -O-,or -S-;
R¹ and R² are the same or different and are independently from each other selected from the group comprising hydrogen, hydroxy, halogen, nitro, cyano, -(CH₂)ₚPO₃(R⁵)₂, -(CH₂)ₚ-NR⁵R⁶, -(CH₂)ₚ-NR⁵COR⁶, -(CH₂)ₚ-NR⁵CSR⁶, -(CH₂)ₚ-NR⁵S(O)R⁶, -(CH₂)ₚ-NR⁵S(O)₂R⁶, -(CH₂)ₚ-NR⁵CONR⁶R⁷, -(CH₂)ₚ-NR⁵COOR⁶, -(CH₂)ₚ-NR⁵C(NH)NR⁶R⁷, -(CH₂)ₚ-NR⁵CSNR⁶R⁷, -(CH₂)ₚ-NR⁵S(O)NR⁶R⁷, -(CH₂)ₚ-NR⁵S(O)₂NR⁶R⁷, -(CH₂)ₚ-COR⁵, -(CH₂)ₚ-CR⁴(OH)-R⁵, -(CH₂)ₚ-CSR⁵, -(CH₂)ₚ-S(O)R⁵ , -(CH₂)ₚ-S(O)(NH)R⁵, -(CH₂)ₚ-S(O)₂R⁵, -(CH₂)ₚ-S(O)₂NR⁵R⁶, -(CH₂)ₚ-S(O)₂N=CH-NR⁵R⁶, -(CH₂)ₚ-SO₃R⁵, -(CH₂)ₚ-CO₂R⁵, -(CH₂)ₚ-CONR⁵R⁶, -(CH₂)ₚ-CSNR⁵R⁶, -(CH₂)ₚ-SR⁵, -(CH₂)ₚ-OR⁵, -CR⁵(OH)-R⁶, -O-(CH₂)ₚ-COR⁵, -O-(CH₂)ₚ-CR⁴(OH)-R⁵, and moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)p-phenyl, -(CH₂)p-C₅-C₆-heteroaryl, -phenylene-(CH₂)p-R ,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl, and wherein 0 to 2 methylene groups of -C₁-C₈-alkyl may be replaced independently of each other by -O-, -S-, -C(=O)-, -SO₂- or-NR⁴-;
R³ is selected from the group comprising halogen, nitro, cyano, -C₁₋C₆-alkylthio, amino,
-NH-(CH₂)ₚ-C₃-C₈-cycloalkyl, benzodioxolyl, -(CH₂)ₚPO₃(R⁵)₂, -NR⁵R⁶, -S(O)(C₁-C₆-alkyl), -S(O)₂(C₁-C₆-alkyl), -CONR⁵R⁶, -COR⁵, -COOR⁵, -NR⁵-(CH₂)ₚ-NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷,
and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)p-phenyl, -(CH₂)ₚ-C₅-C₁₀-heteroaryl, -O-phenyl, -O-C₅-C₁₀-heteroaryl, -phenylene-(CH₂)p-R⁵,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -NH-(CH₂)ₚ-C₃-C₈-cycloalkyl, -C₃-C₈-cycloalkyl, -C₃-C₈-heterocycloalkyl, -(CH₂)ₚ-phenyl, -(CH₂)ₚ-C₅-C₆-heteroaryl, -phenylene-(CH₂)ₚ-R⁵, -NR⁵R⁶, -C₁-C₆-hydroxyalkyl, -C₁-C₆-haloalkyl, -C₁-C₆-haloalkoxy, -C₂-C₆-alkenyl, -C₂-C₆-alkynyl, -C₁-C₆-alkoxy-C₁-C₆-alkyl, -C₁-C₆-alkoxy-C₁-C₆-alkoxy-C₁-C₆-alkyl, -(CH₂)ₚPO₃(R⁵)₂, -(CH₂)ₚ-NR⁵CONR⁶R⁷, -(CH₂)ₚ-S(O)R⁵, -(CH₂)ₚ-S(O)₂R⁵, -(CH₂)ₚ-NR⁵S(O)₂R⁶, -(CH₂)ₚ-S(O)₂NR⁵R⁶, -(CH₂)ₚ-CONR⁵R⁶, -(CH₂)ₚ-NR⁵COR⁶, -(CH₂)ₚ-CR⁴(OH)-R⁵, -O-(CH₂)ₚ-CR⁴(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein phenylene, -C₃-C₈-cydoalkyl, -C₃-C₈-heterocycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃, and wherein 0 to 2 methylene groups of -C₁-C₈-alkyl may be replaced independently of each other by -O-, -S-, -C(=O)-, -SO₂₋or -NR⁴-;
R⁴ is hydrogen or -C₁-C₈-alkyl;
R⁵, R⁶, R⁷ and R⁸ are the same or different and are independently from each other selected from the group comprising hydrogen, benzodioxolyl and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, phenyl, -C₅-C₁₀-heteroaryl,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -NH-(CH₂)ₚ-C₃-C₈-cycloalkyl, -C₃-C₈-cycloalkyl, -C₃-C₈-heterocydoalkyl, -C₁-C₆-hydroxyalkyl, -C₁-C₆-haloalkyl, -C₁-C₆-haloalkoxy, -C₂-C₆-alkenyl, -C₂-C₆-alkynyl, -C₁-C₆-alkoxy-C₁-C₆-alkyl, -C₁-C₆-alkoxy-C₁-C₆-alkoxy-C₁-C₆-alkyl, -NR⁹R¹⁰, -NR⁹CONR¹⁰, -S(O)(C₁-C₆-alkyl), -S(O)₂(C₁-C₆-alkyl), -S(O)₂-phenyl, -NH-S(O)₂(C₁-C₆-alkyl), -NH-S(O)₂-phenyl, -S(O)₂-NH-(C₁-C₆-alkyl), -S(O)₂-NH-phenyl, -C₁-C₆-alkanoyl, -CONR⁹R¹⁰, -COR⁹, -COOR⁹, -(CH₂)p-phenyl, -(CH₂)ₚ-C₅-C₁₀-heteroaryl, -phenylene-(CH₂)p-R⁹, -(CH₂)pPO₃(R⁹)₂,
wherein phenylene, -C₃-C₈-cycloalkyl, -C₃-C₈-heterocycloalkyl, phenyl, -(CH₂)ₚ-phenyl and -(CH₂)ₚ-C₅-C₁₀-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or -NR⁴-;
or
two members selected from the group comprising R⁵, R⁶, R⁷ and R⁸ form a 2- to 8-membered alkylene tether which may be unsubstituted or singly substituted by -(CH₂)ₚ-OH, -(CH₂)ₚ-CN, or -(CH₂)ₚ-NR⁹R¹⁰, and in which 0 to 2 methylene groups may be replaced by -O-, -S-, -(C=O)-, -S(O)₂-, or -NR⁴-;
R⁹, R¹⁰ are independently from each other hydrogen or -C₁-C₈-alkyl;
p is from 0-6;
and solvates, hydrates, N-oxides, isomers and salts thereof.

2. The compound according to claim 1, wherein
A is *-meta*-phenylene- or -C₅-C₆-heteroarylene-;
X is a 2- to 6-membered alkylene tether which is unsubstituted or singly or multiply substituted by substituents selected from the group comprising C₁-C₄-alkoxy, hydroxy, amino, -NH-C₁-C₄-alkyl, -N(C₁-C₄-alkyl)₂, or C₁-C₄-alkyl, which itself may be unsubstituted or singly or multiply substituted by C₁-C₄-alkoxy, hydroxy, amino, -NH-C₁-C₄-alkyl, or -N(C₁-C₄-alkyl)₂;
Y is -NR⁴- or -O-;
R¹ and R² are the same or different and are independently from each other selected from the group comprising hydrogen, hydroxy, halogen, nitro, cyano, -(CH₂)ₚ-NR⁵R⁶, -(CH₂)ₚ-NR⁵COR⁶, -(CH₂)ₚ-NR⁵S(O)₂R⁶, -(CH₂)ₚ-NR⁵CONR⁶R⁷, -(CH₂)ₚ-NR⁵COOR⁶, -(CH₂)ₚ-COR⁵, -(CH₂)ₚ-CR⁴(OH)-R⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-CO₂R⁵, -(CH₂)ₚ-CONR⁵R⁶, -(CH₂)ₚ-OR⁵, -CR⁵(OH)-R⁶, -O-(CH₂)ₚ-COR⁵, -O-(CH₂)ₚ-CR⁴(OH)-R⁵,
and moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)ₚ-phenyl, -(CH₂)ₚ-C₅-C₆-heteroaryl,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl, and wherein 0 to 2 methylene groups of -C₁-C₈-alkyl may be replaced independently of each other by -O-, -S-, -C(=O)-, -S(O)₂- or -NR⁴-;
R³ is selected from the group comprising halogen, nitro, cyano, -C₁₋C₆-alkylthio, amino, benzodioxolyl,
-NR⁵R⁶, -S(O)₂(C₁-C₆-alkyl), -CONR⁵R⁶, -COR⁵, -COOR⁵, -NR⁵-(CH₂)ₚ-NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷,
and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)ₚ-phenyl, -(CH₂)ₚ-C₅-C₁₀-heteroaryl, -O-phenyl, -O-C₅-C₁₀-heteroaryl, -phenylene-(CH₂)ₚ-R⁵,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -C₃-C₈-cycloalkyl, -C₃-C₈-heterocycloalkyl, -(CH₂)ₚ-phenyl, -(CH₂)ₚ-C₅-C₆-heteroaryl, -phenylene-(CH₂)ₚ-R⁵, -NR⁵R⁶, -C₁-C₆-hydroxyalkyl, -C₁-C₆-haloalkyl, -C₁-C₆-haloalkoxy, -C₂-C₆-alkenyl, -C₂-C₆-alkynyl, -C₁-C₆-alkoxy-C₁-C₆-alkyl, -(CH₂)ₚ-NR⁵CONR⁶R⁷, -(CH₂)ₚ-S(O)₂R⁵, -(CH₂)ₚ-NR⁵S(O)₂R⁶, -(CH₂)ₚ-S(O)₂NR⁵R⁶, -(CH₂)ₚ-CONR⁵R⁶, -(CH₂)ₚ-NR⁵COR⁶, -(CH₂)ₚ-CR⁴(OH)-R⁵, -O-(CH₂)ₚ-CR⁴(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein phenylene, -C₃-C₈-cycloalkyl, -C₃-C₈-heterocycloalkyl, -(CH₂)ₚ-phenyl and -(CH₂)ₚ-C₅-C₁₀-heteroaryl are unsubstituted or singly or multiply substituted independently from each other with halogen, hydroxy, -C₁-C₆-alkyl, -C₁-C₆-alkoxy, -CF₃ and/or -OCF₃, and wherein 0 to 2 methylene groups of -C₁-C₈-alkyl may be replaced independently of each other by -O-, -S-, -C(=O)-, -S(O)₂- or -NR⁴-;
R⁴ is hydrogen or -C₁-C₈-alkyl;
R⁵, R⁶, R⁷ and R⁸ are the same or different and are independently from each other selected from the group comprising hydrogen, benzodioxolyl and moieties, said moieties being selected from the group comprising -C₁-C₈-alkyl, -C₃-C₈-cydoalkyl, -C₃-C₈-heterocydoalkyl, phenyl, -C₅-C₁₀-heteroaryl,
wherein said moieties are unsubstituted or singly or multiply substituted independently from each other with hydroxy, halogen, -C₁-C₆-alkoxy, -C₁-C₆-alkylthio, amino, cyano, -C₁-C₈-alkyl, -C₃-C₈-cycloalkyl, -C₃-C₈-heterocycloalkyl, -C₁-C₆-hydroxyalkyl, -C₁-C₆-haloalkyl, -C₁-C₆-haloalkoxy, -NR⁹R¹⁰, -NR⁹CONR¹⁰, -S(O)₂(C₁-C₆-alkyl), -S(O)₂-phenyl, -NH-S(O)₂(C₁-C₆-alkyl), -NH-S(O)₂-phenyl, -S(O)₂-NH-(C₁-C₆-alkyl), -S(O)₂-NH-phenyl, -C₁-C₆-alkanoyl, -CONR⁹R¹⁰, -COR⁹, -COOR⁹, -(CH₂)ₚ-phenyl, -(CH₂)ₚ-C₅-C₆-heteroaryl, -phenylene-(CH₂)ₚ-R⁹,
wherein phenylene, -C₃-C₈-cycloalkyl, -C₃-C₈-heterocycloalkyl, phenyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or -NR⁴-;
or
two members selected from the group comprising R⁵, R⁶, R⁷ and R⁸ form a 2- to 8-membered alkylene tether which may be unsubstituted or singly substituted by -(CH₂)ₚ-OH or -(CH₂)ₚ-NR⁹R¹⁰, and in which 0 to 2 methylene groups may be replaced by -O- or -NR⁴-;
R⁹, R¹⁰ are independently from each other hydrogen or -C₁-C₈-alkyl;
p is from 0 - 6.

3. The compound according to claim 1 or 2, wherein
A is -meta-phenylene-;
X is an unsubstituted 4- to 5-membered alkylene tether;
Y is -NR⁴- or -O-;
R¹ and R² are the same or different and are independently from each other selected from the group comprising hydrogen, hydroxy, halogen, nitro, cyano, -(CH₂)ₚ-NR⁵R⁶ , -(CH₂)ₚ-NHCOR⁶, -(CH₂)ₚ-NHS(O)₂R⁶, -(CH₂)ₚ-NHCONR⁶R⁷, -(CH₂)ₚ-NHCOOR⁶ , -(CH₂)ₚ-COR⁵, -(CH₂)ₚ-CH(OH)-R⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-CO₂R⁵, -(CH₂)ₚ-CONR⁵R⁶, -(CH₂)ₚ-OR⁵, -CR⁵(OH)-R⁶, -O-(CH₂)ₚ-COR⁵, -O-(CH₂)ₚ-CH(OH)-R⁵
and moieties being selected from the group comprising -C₁-C₈-alkyl, -(CH₂)p-phenyl, -(CH₂)ₚ-C₅-C₆-heteroaryl,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl;
R³ is selected from the group comprising halogen, cyano, amino, benzodioxolyl, -NR⁵R⁶, -CONR⁵R⁶, -COR⁵, -NR⁵-(CH₂)ₚ₋NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷,
and moieties, said moieties being selected from the group comprising -C₁-C₈-alkyl, -C₂-C₈-alkenyl, -C₂-C₈-alkynyl, -C₃-C₈-cycloalkyl, -C₃-C₈-heterocycloalkyl, -(CH₂)ₚ-phenyl, -(CH₂)ₚ-C₅-C₁₀-heteroaryl,
wherein said moieties are unsubstituted or singly or multiply substituted independently from each other with hydroxy, halogen, -C₁-C₆-alkoxy, amino, cyano, -C₁-C₆-alkyl, -C₃-C₈-cydoalkyl, -C₃-C₈-heterocycloalkyl, -(CH₂)p-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷ , -(CH₂)ₚ-S(O)₂R⁵, -(CH₂)ₚ-NHS(O)₂R⁶ , -(CH₂)ₚ-S(O)₂NR⁵R⁶, -(CH₂)ₚ-CONR⁵R⁶ , -(CH₂)ₚ-NHCOR⁶ , -(CH₂)ₚ-CH(OH)-R⁵, -O-(CH₂)ₚ-CH(OH)-R⁵, -(CH₂)ₚ-COR⁵, -O-(CH₂)ₚ-COR , -(CH₂)ₚ-COOR⁸, -O-(CH₂)ₚ-R⁵,
wherein -C₃-C₈-cycloalkyl, -C₃-C₈-heterocycloalkyl, -(CH₂)p-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃, and wherein 0 to 2 methylene groups of -C₁-C₈-alkyl may be replaced independently of each other by -0-, -S-, -C(=O)-, -S(O)₂- or -NR⁴-.
R⁴ is hydrogen or -C₁-C₈-alkyl;
R⁵, R⁶, R⁷ and R⁸ are the same or different and are independently from each other selected from the group comprising hydrogen, benzodioxolyl and moieties, said moieties being selected from the group comprising -C₁-C₈-alkyl, -C₃-C₈-cycloalkyl, -C₃-C₈-heterocycloalkyl, phenyl, -C₅-C₁₀-heteroaryl,
wherein said moieties are unsubstituted or singly or multiply substituted independently from each other with hydroxy, halogen, -C₁-C₆-alkoxy, amino, cyano, -C₁-C₈-alkyl, -C₃-C₈-cydoalkyl, -C₁-C₆-hydroxyalkyl, -C₁-C₆-haloalkyl, -C₁-C₆-haloalkoxy, -NR⁹R¹⁰, -NR⁹CONR¹⁰, -S(O)₂(C₁-C₆-alkyl), -S(O)₂-phenyl, -NH-S(O)₂(C₁-C₆-alkyl), -NH-S(O)₂-phenyl, -C₁-C₆-alkanoyl, -CONR⁹R¹⁰, -COR⁹, -COOR⁹, -(CH₂)ₚ-phenyl, -(CH₂)ₚ-C₅-C₆-heteroaryl,
wherein -C₃-C₈-cydoalkyl, phenyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃,
and wherein 0 to 2 methylene groups of -C₁-C₈-alkyl may be replaced independently of each other by -O-, -S-, -C(=O)-, -S(O)₂- or -NR⁴-;
or
two members selected from the group comprising R⁵, R⁶, R⁷ and R⁸ form a 3- to 7-membered alkylene tether which may be unsubstituted or singly substituted by -(CH₂)ₚ-OH or -(CH₂)p-NR⁹R¹⁰, and in which 0 to 2 methylene groups may be replaced by -O- or -NR⁴-;
R⁹, R¹⁰ are independently from each other hydrogen or -C₁-C₈-alkyl;
p is from 0 - 4.

4. The compound according to any one of the preceding claims, wherein
R⁴ is hydrogen, methyl, or ethyl.

5. The compound according to any one of the preceding claims, wherein
R⁵, R⁶, R⁷ and R⁸ are the same or different and are independently from each other selected from the group comprising hydrogen and moieties, said moieties being selected from the group comprising -C₃-C₈₋cycloalkyl, -C₃-C₈-heterocycloalkyl, -C₁-C₈-alkyl, phenyl and -C₅-C₆-heteroaryl,
wherein said moieties are unsubstituted or singly or multiply substituted independently from each other with hydroxy, halogen, -C₁-C₆-alkoxy, amino, cyano, -C₁-C₆-alkyl, -C₁-C₆-hydroxyalkyl, -C₁-C₆-haloalkyl, -C₁-C₆-haloalkoxy, -NR⁹R¹⁰, -CONR⁹R¹⁰, -COR⁹,
or
two members selected from the group comprising R⁵, R⁶, R⁷ and R⁸ form a 3- to 7-membered alkylene tether which may be unsubstituted or singly substituted by -(CH₂)ₚ-OH or -(CH₂)ₚ-NR⁹R¹⁰, and in which 0 to 2 methylene groups may be replaced by -O- or -NR⁴-.

6. The compound according to any one of the preceding claims, wherein
R¹ and R² are the same or different and are independently from each other selected from the group comprising hydrogen, -(CH₂)ₚ-NHCOR⁶, -(CH₂)ₚ-NHS(O)₂R⁶, -(CH₂)ₚ-NHCONR⁶R⁷, -(CH₂)ₚ-NHCOOR⁶, -(CH₂)ₚ-COR⁵, -(CH₂)ₚ-CH(OH)-R⁵, -(CH₂)ₚ-S(O)(NH)R⁵, -(CH₂)ₚ-S(O)₂R⁵, -(CH₂)ₚ-S(O)₂NR⁵R⁶ -(CH₂)ₚ-CO₂R⁵, -(CH₂)ₚ-CONR⁵R⁶, -CR⁵(OH)-R⁶, -O-(CH₂)ₚ-COR⁵, -O-(CH₂)ₚ-CH(OH)-R⁵.

7. The compound according to any one of the preceding claims, wherein
R¹ and R² are the same or different and are independently from each other selected from the group comprising hydrogen, -(CH₂)ₚ-NHCOR⁶, -(CH₂)ₚ-NHS(O)₂R⁶, -(CH₂)ₚ-NHCONR⁶R⁷, -(CH₂)ₚ-NHCOOR⁶, -(CH₂)ₚ-COR⁵, -(CH₂)ₚ-S(O)(NH)R⁵, -(CH₂)ₚ₋S(O)R⁵, -(CH₂)ₚ-S(O)₂NR⁵R⁶, -(CH₂)ₚ-CONR⁵R⁶, -0-(CH₂)ₚ-COR⁵.

8. The compound according to any one of the preceding claims, wherein
R¹ and R² are the same or different and are independently from each other selected from the group comprising hydrogen, -(CH₂)ₚ-NHCOR⁶, -(CH₂)ₚ-NHS(O)₂R⁶, -(CH₂)ₚ-NHCONR⁶R⁷.

9. The compound according to any one of the claims 1 - 5, wherein
R¹ and R² are the same or different and are independently from each other selected from the group comprising -C₁-C₈-alkyl, -(CH₂)ₚ-phenyl, -(CH₂)ₚ-C₅-C₆-heteroaryl,
wherein said moieties are unsubstituted or singly or multiply substituted independently from each other with hydroxy, halogen, phenyl, -NR⁵R⁶, cyano, -C₁-C₄-alkyl, -C₁-C₄-alkoxy, -C₁-C₄-haloalkyl, -C₁-C₄-haloalkoxy, or -C₁-C₄-hydroxyalkyl.

10. The compound according to any one of claims 1-5, wherein
R³ is selected from the group comprising halogen, cyano, amino, benzodioxolyl, -NR⁵R⁶, -CONR⁵R⁶, -COR⁵, -NR⁵-(CH₂)ₚ-NR⁶CONR⁷R⁸, -NR⁵-(CH₂)ₚ-NR⁶S(O)₂R⁷, -NR⁵-(CH₂)ₚ-NR⁶COR⁷, -NR⁵-(CH₂)ₚ-CONR⁶R⁷.

11. The compound according to any one of claims 1 - 5, wherein
R³ is selected from the group comprising -NR⁵R⁶, -CONR⁵R⁶, -COR⁵, -NR⁵-(CH₂)ₚ-NR⁶CONR⁷R⁸, -NR⁵ -(CH₂)ₚ-NR⁶S(O)₂R⁷, -NR⁵-(CH₂)ₚ-NR⁶COR⁷, -NR⁵-(CH₂)ₚ-CONR⁶R⁷.

12. The compound according to any one of claims 1 - 5, wherein
R³ is selected from the group comprising halogen, cyano, amino.

13. The compound according any one of claims 1 - 5, wherein
R³ is benzodioxolyl.

14. The compound according to any one of the claims 1 - 5, wherein
R³ is selected from the group comprising -C₁-C₈-alkyl, -C₂-C₈-alkenyl, and -C₂-C₈-alkynyl, wherein said moieties are unsubstituted or singly or multiply substituted independently from each other with hydroxy, halogen, -C₁-C₆-alkoxy, amino, cyano, -C₁-C₆-alkyl, -C₃-C₈-cycloalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -(CH₂)ₚ-COOR⁵, -O-(CH₂)ₚ-R⁵, wherein -C₃-C₈-cycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

15. The compound according to any one of claims 1 - 5, wherein
R³ is selected from the group comprising -C₁-C₈-alkyl and -C₂-C₈-alkynyl, wherein said moieties are unsubstituted or singly or multiply substituted independently from each other with halogen, -C₁-C₆-alkoxy, C₁-C₆-alkyl, -(CH₂)ₚ-phenyl, -(CH₂)ₚ-C₅-C₆-heteroaryl, -NR⁵R⁶, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶. -(CH₂)ₚ-CONR⁵R⁶, -(CH₂)ₚ-NHCOR⁶, -(CH₂)ₚ-CH(OH)-R⁵, -(CH₂)ₚ-COR⁵,
wherein -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

16. The compound according to any one of the claims 1 - 5, wherein
R³ is selected from the group comprising -C₃-C₈-cycloalkyl and -C₃₋C₈-heterocycloalkyl, and is unsubstituted or singly or multiply substituted independently from each other with hydroxy, halogen, -C₁-C₆-alkoxy, amino, cyano, -C₁-C₆-alkyl, -C₃-C₈-cydoalkyl, -C₃-C₈-heterocydoalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein -C₃-C₈-cycloalkyl, -C₃-C₈-heterocycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

17. The compound according to any one of claims 1 - 5, wherein
R³ is selected from the group comprising cyclopentyl, cyclohexyl, piperazinyl, piperidinyl, 1,4-diazepanyl and pyrrolidinyl, wherein said moieties are unsubstituted or singly or multiply substituted independently from each other with hydroxy, -C₁-C₆-alkoxy, cyano, -C₁-C₆-alkyl, -C₃-C₈-cycloalkyl, -C₃-C₈-heterocycloalkyl, -NR⁵R⁶, -C₁-C₆-hydroxyalkyl, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

18. The compound according to any one of claims 1-5, wherein
R³ is selected from the group comprising piperazinyl, piperidinyl, and pyrrolidinyl, wherein said moieties are unsubstituted or singly or multiply substituted independently from each other with -C₁-C₆-alkyl, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

19. The compound according to any one of claims 1-5, wherein
R³ is piperazinyl and is unsubstituted or singly or multiply substituted independently from each other with -C₁-C₆-alkyl, -(CH₂)ₚ-S(O)₂R⁵, -(CH₂)ₚ-CONR⁵R⁶, -(CH₂)ₚ-CH(OH)-R⁵, -(CH₂)ₚ-COR⁵.

20. The compound according to any one of claims 1 - 5, wherein
R³ is selected from the group comprising -(CH₂)ₚ-phenyl and -(CH₂)ₚ-C₅-C₁₀-heteroaryl,
wherein said moieties are unsubstituted or singly or multiply substituted independently from each other with hydroxy, halogen, -C₁-C₆-alkoxy, amino, cyano, -C₁-C₆-alkyl, -C₃-C₈-cycloalkyl, -C₃-C₈-heterocycloalkyl, -(CH₂)ₚ-phenyl, -(CH₂)ₚ-C₅-C₆-heteroaryl, -NR⁵R⁶, -C₁-C₆-hydroxyalkyl, -C₁-C₆-haloalkyl, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶ -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -(CH₂)ₚ-COOR⁵, -O-(CH₂)ₚ-R⁵,
wherein -C₃-C₈-cycloalkyl, -C₃-C₈-heterocycloalkyl, -(CH₂)ₚ-phenyl and -(CH₂)ₚ-C₅-C₆-heteroaryl are unsubstituted or singly or multiply substituted independently from each other with halogen, hydroxy, -C₁-C₆-alkyl, -C₁-C₆-alkoxy, -CF₃ and/or -OCF₃.

21. The compound according to any one of claims 1-5, wherein
R³ is selected from the group comprising -(CH₂)ₚ-phenyl and -(CH₂)ₚ-C₅-C₆-heteroaryl,
wherein said moieties are unsubstituted or singly or multiply substituted independently from each other with halogen, -C₁-C₆-alkoxy, cyano, -C₁-C₆-alkyl, -NR⁵R⁶, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)ₚ-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

22. The compound according to any one of claims 1-5, wherein
R³ is selected from the group comprising phenyl and -C₅-C₆-heteroaryl,
wherein said moieties are unsubstituted or singly or multiply substituted independently from each other with halogen, -C₁-C₆-alkoxy, cyano, -C₁-C₆-alkyl, -NR⁵R⁶, -C₁-C₆-haloalkoxy, -(CH₂)ₚ-NHCONR⁶R⁷, -(CH₂)ₚ-S(O)₂R⁵, -(CH₂)ₚ-NHS(O)₂R⁶, -(CH₂)ₚ-S(O)₂NR⁵R⁶, -(CH₂)p-CONR⁵R⁶, -(CH₂)ₚ-NHCOR⁶, -(CH₂)ₚ-CH(OH)-R⁵, -O-(CH₂)ₚ-CH(OH)-R⁵, -(CH₂)ₚ-COR⁵, -O-(CH₂)ₚ-COR⁵, -O-(CH₂)ₚ-R⁵.

23. The compound according to any one of claims 1 - 5, wherein
R³ is phenyl and is unsubstituted or singly or multiply substituted independently from each other with halogen, -C₁-C₆-alkoxy, cyano, -C₁-C₆-alkyl, -NR⁵R⁶, -C₁-C₆-haloalkoxy, -(CH₂)ₚ-NHCONR⁶R⁷, -(CH₂)ₚ-NHS(O)₂R⁶, -(CH₂)ₚ-CONR⁵R⁶, -(CH₂)ₚ-NHCOR⁶, -(CH₂)ₚ-COR⁵, -O-(CH₂)ₚ-COR⁵.

24. A method of preparing the compound of Formula I according to any one of claims 1 - 23, comprising the following method steps:
A) a disulfide of Formula A is transferred into a sulfoximine of Formula B 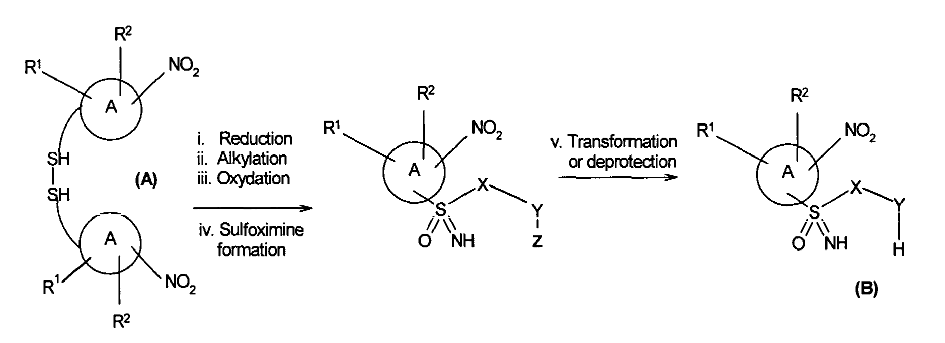 in which A, Y, R¹ and R² have the same meaning as in Formula I and Z is a protecting or activating group;
B) the sulfoximine of Formula B is transferred into a sulfoximine of Formula C 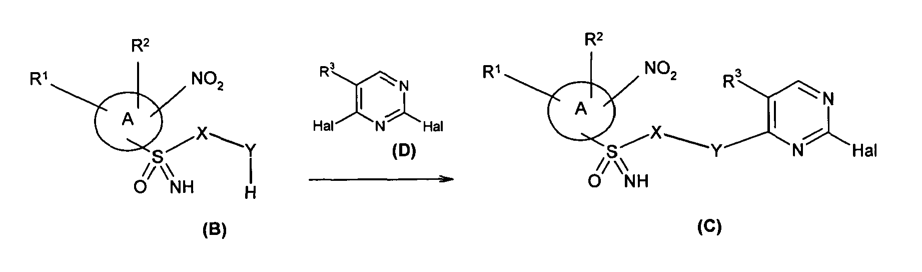 in which A, X, Y, R¹, R², and R³ have the same meaning as in Formula I, and wherein Hal is halogen; and
C) the sulfoximine of Formula C is transferred into a macrocyclic sulfoximine of Formula I 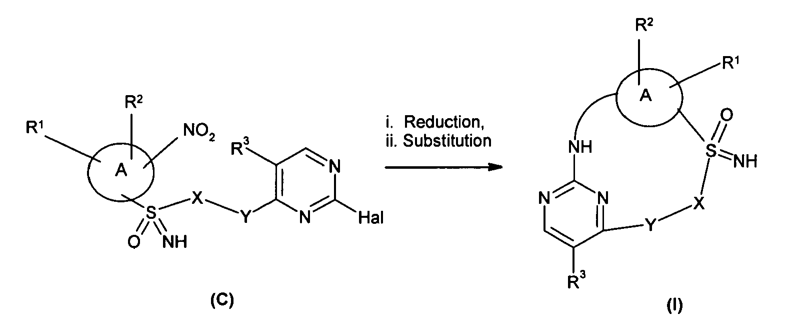 in which A, X, Y, R¹, R², and R³ and Hal have the same meaning as in Formula I.

25. A pharmaceutical composition which comprises the compounds of the Formula I according to any one of claims 1 - 23 or an in vivo hydrolysable ester thereof, and one or more pharmaceutically-acceptable diluent or carrier.

26. A use of the compound of the Formula I according to any one of claims 1-23 for manufacturing a pharmaceutical composition for the treatment of diseases of dysregulated vascular growth or of diseases which are accompanied with dysregulated vascular growth.

27. The use according to claim 26, wherein the diseases are retinopathy, other angiogenesis dependent diseases of the eye, rheumatoid arthritis, and other inflammatory diseases associated with angiogenesis.

28. The use according to claim 27, wherein the other angiogenesis dependent diseases of the eye are cornea transplant rejection, age-related macular degeneration.

29. The use according to claim 27, wherein the inflammatory diseases associated with angiogenesis are psoriasis, delayed type hypersensitivity, contact dermatitis, asthma, multiple sclerosis, restenosis, pulmonary hypertension, stroke, and diseases of the bowel.

30. The use according to claim 26, wherein the diseases are coronary and peripheral artery disease.

31. The use according to claim 26, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorption and for benign proliferating diseases like myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

32. The use according to claim 26, wherein the disease is a solid tumour and/or metastases thereof.

33. A method for treating a disease of dysregulated vascular growth or diseases which are accompanied with dysregulated vascular growth.

34. The method according to claim 33, wherein the diseases are retinopathy, other angiogenesis dependent diseases of the eye rheumatoid arthritis, and other inflammatory diseases associated with angiogenesis.

35. The method according to claim 34, wherein the angiogenesis dependent diseases of the eye are cornea transplant rejection, age-related macular degeneration.

36. The method according to claim 34, wherein the inflammatory diseases associated with angiogenesis are psoriasis, delayed type hypersensitivity, contact dermatitis, asthma, multiple sclerosis, restenosis, pulmonary hypertension, stroke, and diseases of the bowel.

37. The method according to claim 33, wherein the diseases are coronary and peripheral artery disease.

38. The method according to claim 33, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorption and for benign proliferating diseases like myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

39. The method according to claim 33, wherein the disease is a solid tumour and/or metastases thereof.
